# EUROPEAN PATENT APPLICATION

(11) **EP 2 383 342 A1**
(43) Date of publication of application: **02.11.2011**
(21) Application number: 10182762.4
(22) Date of filing: 07.04.1999
(51) Int. Cl.: C12N 15/31, C07K 14/35, C07K 19/00, C12P 21/02, A61K 39/04

(54) **Fusion proteins of mycobacterium tuberculosis antigens and their uses**

(30) Priority: 07.04.1998 US 56556; 30.12.1998 US 223040
(62) Divisional of application: 99916513.7
(71) Applicant: CORIXA CORPORATION, Wilmington, DE 19808 (US)
(72) Inventor: Skeiky, Yasir A W, Maryland, WA 20904 (US); Alderson, Mark, Bainbridge Island, WA 98110 (US); Campos-Neto, Antionio, Boston, MA 02115-3799 (US)
(74) Representative: Goodall, Scott

(57) **Abstract**

The present invention relates to fusion proteins containing *Mycobacterium tuberculosis* antigens and methods for their use in the diagnosis, treatment and prevention of tuberculosis infection.

## Description

### 1. INTRODUCTION

The present invention relates to fusion proteins containing at least two *Mycobacterium tuberculosis* antigens. In particular, it relates to bi- fusion proteins which contain two individual *M. tuberculosis* antigens, tri- fusion proteins which contain three *M. tuberculosis* antigens, tetra-fusion proteins which contain four *M. tuberculosis* antigens, and penta-fusion proteins which contain five *M*. *tuberculosis* antigens, and methods for their use in the diagnosis, treatment and prevention of tuberculosis infection.

### 2. BACKGROUND OF THE INVENTION

Tuberculosis is a chronic infectious disease caused by infection with *M*. *tuberculosis.* It is a major disease in developing countries, as well as an increasing problem in developed areas of the world, with about 8 million new cases and 3 million deaths each year. Although the infection may be asymptomatic for a considerable period of time, the disease is most commonly manifested as an acute inflammation of the lungs, resulting in fever and a nonproductive cough. If untreated, serious complications and death typically result.

Although tuberculosis can generally be controlled using extended antibiotic therapy, such treatment is not sufficient to prevent the spread of the disease. Infected individuals may be asymptomatic, but contagious, for some time. In addition, although compliance with the treatment regimen is critical, patient behavior is difficult to monitor. Some patients do not complete the course of treatment, which can lead to ineffective treatment and the development of drug resistance.

In order to control the spread of tuberculosis, effective vaccination and accurate early diagnosis of the disease are of utmost importance. Currently, vaccination with live bacteria is the most efficient method for inducing protective immunity. The most common Mycobacterium employed for this purpose is *Bacillus* Calmette-Guerin (BCG), an avirulent strain of *M. bovis.* However, the safety and efficacy of BCG is a source of controversy and some countries, such as the United States, do not vaccinate the general public with this agent.

Diagnosis of tuberculosis is commonly achieved using a skin test, which involves intradermal exposure to tuberculin PPD (protein-purified derivative). Antigen-specific T cell responses result in measurable induration at the injection site by 48-72 hours after injection, which indicates exposure to Mycobacterial antigens. Sensitivity and specificity have, however, been a problem with this test, and individuals vaccinated with BCG cannot be distinguished from infected individuals.

While macrophages have been shown to act as the principal effectors of *M*. *tuberculosis* immunity, T cells are the predominant inducers of such immunity. The essential role of T cells in protection against *M. tuberculosis* infection is illustrated by the frequent occurrence *of M. tuberculosis* in Acquired Immunodeficiency Syndrome patients, due to the depletion of CD4⁺ T cells associated with human immunodeficiency virus (HIV) infection. Mycobacterium-reactive CD4⁺ T cells have been shown to be potent producers of gamma-interferon (IFN-γ), which, in turn, has been shown to trigger the anti-mycobacterial effects of macrophages in mice. While the role of IFN-γ in humans is less clear, studies have shown that 1,25-dihydroxy-vitamin D3, either alone or in combination with IFN-γ or tumor necrosis factor-alpha, activates human macrophages to inhibit *M. tuberculosis* infection. Furthermore, it is known that IFN-γ stimulates human macrophages to make 1,25-dihydroxy-vitamin D3. Similarly, interleukin-12 (IL-12) has been shown to play a role in stimulating resistance to *M*. *tuberculosis* infection. For a review of the immunology of *M. tuberculosis* infection, see Chan and Kaufinann, 1994, Tuberculosis: Pathogenesis, Protection and Control, Bloom (ed.), ASM Press, Washington, DC.

Accordingly, there is a need for improved vaccines, and improved methods for diagnosis, preventing and treating tuberculosis.

### 3. SUMMARY OF THE INVENTION

The present invention relates to fusion proteins of *M. tuberculosis* antigens. In particular, it relates to fusion polypeptides that contain two or more *M. tuberculosis* antigens, polynucleotides encoding such polypeptides, methods of using the polypeptides and polynucleotides in the diagnosis, treatment and prevention *of M. tuberculosis* infection.

The present invention is based, in part, on the inventors' discovery that polynucleotides which contain two to five *M. tuberculosis* coding sequences produce recombinant fusion proteins that retain the immunogenicity and antigenicity of their individual components. The fusion proteins described herein induced both T cell and B cell responses, as measured by T cell proliferation, cytokine production, and antibody production. Furthermore, a fusion protein was used as an immunogen with adjuvants in *vivo* to elicit both cell-mediated and humoral immunity to *M. tuberculosis.* Additionally, a fusion protein was made by a fusion construct and used in a vaccine formulation with an adjuvant to afford long-term protection in animals against the development of tuberculosis. The fusion protein was a more effective immunogen than a mixture of its individual protein components.

In a specific embodiment of the invention, the isolated or purified *M. tuberculosis* polypeptides of the invention may be formulated as pharmaceutical compositions for administration into a subject in the prevention and/or treatment of *M*. *tuberculosis* infection. The immunogenicity of the fusion protein may be enhanced by the inclusion of an adjuvant.

In another aspect of the invention, the isolated or purified polynucleotides are used to produce recombinant fusion polypeptide antigens *in vitro.* Alternatively, the polynucleotides may be administered directly into a subject as DNA vaccines to cause antigen expression in the subject, and the subsequent induction of an anti-*M*. *tuberculosis* immune response.

It is also an object of the invention that the polypeptides be used in *in vitro* assays for detecting humoral antibodies or cell-mediated immunity against *M. tuberculosis* for diagnosis of infection or monitor of disease progression. Additionally, the polypeptides may be used as an *in vivo* diagnostic agent in the form of an intradermal skin test. Alternatively, the polypeptides may be used as immunogens to generate anti-*M*. *tuberculosis* antibodies in a non-human animal. The antibodies can be used to detect the target antigens *in vivo* and *in vitro.*

### 4. BRIEF DESCRIPTION OF THE DRAWINGS

- Figure 1A and 1B.: The nucleotide sequence (SEQ ID NO:1) and amino acid sequence (SEQ ID NO:2) of tri-fusion protein Ra12-TbH9-Ra35 (designated Mtb32A).
- Figure 2 :: The nucleotide sequence (SEQ ID NO:3) and amino acid sequence (SEQ ID NO:4) of tri-fusion protein Erd14-DPV-MTI (designated Mtb39A).
- Figure 3A - 3D:: The nucleotide sequence (SEQ ID NO:5) and amino acid sequence (SEQ ID NO:6) of tri-fusion protein TbRa3-38kD-Tb38-1.
- Figure 4A - 4D:: The nucleotide sequence (SEQ ID NO:7) and amino acid sequence (SEQ ID NO:8) of bi-fusion protein TbH9-Tb38-1.
- Figure 5A - 5J:: The nucleotide sequence (SEQ ID NO:9) and amino acid sequence (SEQ ID NO: 10) of tetra-fusion protein TbRa3-38kD-Tb38-1-DPEP (designated TbF-2).
- Figure 6A and 6B:: The nucleotide sequence (SEQ ID NO:11) and amino acid sequence (SEQ ID NO: 12) of penta-fusion protein Erd14-DPV-MTI-MSL-MTCC2 (designated Mtb88f).
- Figure 7A and 7B:: The nucleotide sequence (SEQ ID NO: 13) and amino acid sequence (SEQ ID NO: 14) of tetra-fusion protein Erd14-DPV-MTI-MSL (designated Mtb46f).
- Figure 8A and 8B:: The nucleotide sequence (SEQ ID NO:15) and amino acid sequence (SEQ ID NO:16) of tetra-fusion protein DPV-MTI-MSL-MTCC2 (designated Mtb71f).
- Figure 9A and 9B:: The nucleotide sequence (SEQ ID NO:17) and amino acid sequence (SEQ ID NO:18) of tri-fusion protein DPV-MTI-MSL (designated Mtb31f).
- Figure 10A and 10B:: The nucleotide sequence (SEQ ID NO:19) and amino acid sequence (SEQ ID NO:20) of tri-fusion protein TbH9-DPV-MTI (designated Mtb61f).
- Figure 11A and 11B:: The nucleotide sequence (SEQ ID NO:21) and amino acid sequence (SEQ ID NO:22) of tri-fusion protein Erd14-DPV-MTI (designated Mtb36f).
- Figure 12A and 12B:: The nucleotide sequence (SEQ ID NO:23) and amino acid sequence (SEQ ID NO:24) of bi-fusion protein TbH9-Ra35 (designated Mtb59f).
- Figure 13A and 13B:: The nucleotide sequence (SEQ ID NO:25) and amino acid sequence (SEQ ID NO:26) of bi-fusion protein Ra12-DPPD (designated Mtb24).
- Figure 14A-14F:: T cell proliferation responses of six PPD+ subjects when stimulated with two fusion proteins and their individual components.
- Figure 15A-15F:: IFN-γ production of six PPD+ subjects when stimulated with two fusion proteins and their individual components.
- Figure 16A-16F:: T cell proliferation of mice immunized with a fusion protein or its individual components and an adjuvant.
- Figure 17:: IFN-γ production of mice immunized with a fusion protein or its individual components and an adjuvant.
- Figure 18:: IL-4 production of mice immunized with a fusion protein or its individual components and an adjuvant.
- Figure 19A-19F:: Serum antibody concentrations of mice immunized with a fusion protein or its individual components and an adjuvant.
- Figure 20A-20C:: Survival of guinea pigs after aerosol challenge of *M. tuberculosis.* Fusion proteins, Mtb32A and Mtb39A, were formulated in adjuvant SBAS1c (20A), SBAS2 (20B) or SBAS7 (20C), and used as an immunogen in guinea pigs prior to challenge with bacteria. BCG is the positive control.
- Figure 21A and 21B:: Stimulation of proliferation and IFN-γ production in TbH9-specific T cells by the fusion protein TbH9-Tb38-1.
- Figure 22A and 22B:: Stimulation of proliferation and IFN-γ production in Tb38-1-specific T cells by the fusion protein TbH9-Tb38-1.
- Figure 23A and 23B:: Stimulation of proliferation and IFN-γ production in T cells previously shown to respond to both TbH-9 and Tb38-1 antigens by the fusion protein TbH9-Tb38-1.

### 5. DETAILED DESCRIPTION OF THE INVENTION

The present invention relates to antigens useful for the treatment and prevention of tuberculosis, polynucleotides encoding such antigens, and methods for their use. The antigens of the present invention are fusion polypeptides *of M. tuberculosis* antigens and variants thereof. More specifically, the antigens of the present invention comprise at least two polypeptides *of M. tuberculosis* that are fused into a larger fusion polypeptide molecule. The antigens of the present invention may further comprise other components designed to enhance the immunogenicity of the antigens or to improve these antigens in other aspects, for example, the isolation of these antigens through addition of a stretch of histidine residues at one end of the antigen.

### 5.1. M. TUBERCULOSIS SPECIFIC ANTIGENS

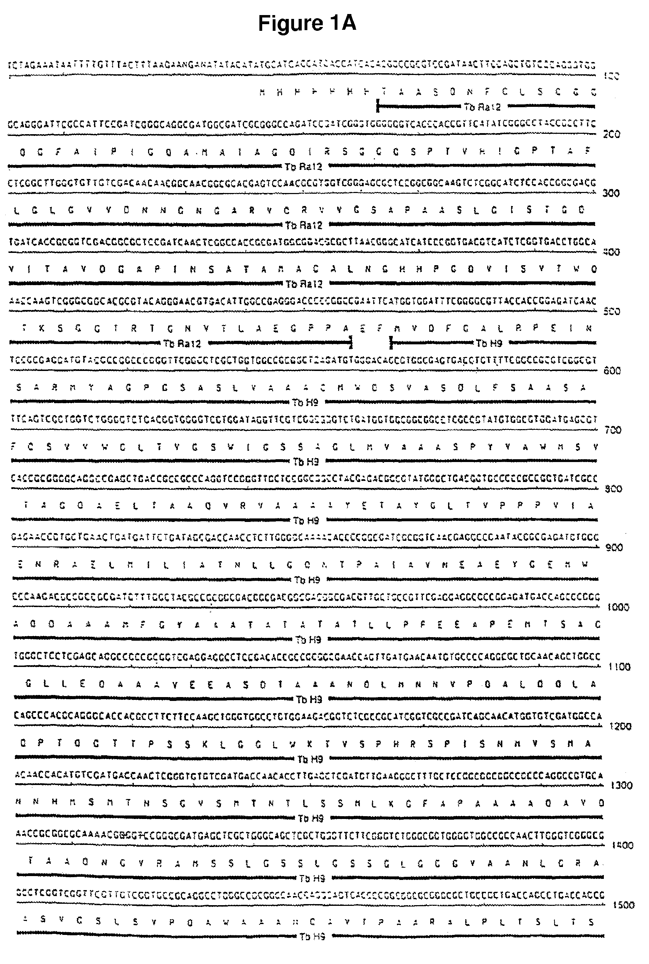

The antigens of the present invention are exemplified in Figures 1A through 13B, including homologues and variants of those antigens. These antigens may be modified, for example, by adding linker peptide sequences as described below. These linker peptides may be inserted between one or more polypeptides which make up each of the fusion proteins presented in Figures 1A through 13B. Other antigens of the present invention are antigens described in Figures 1A through 13B which have been linked to a known antigen *of M. tuberculosis,* such as the previously described 38 kD (SEQ ID NO:27) antigen (Andersen and Hansen,1989, Infect. Immun. 57:2481-2488; Genbank Accession No. M30046).

### 5.2. IMMUNOGENICITY ASSAYS

Antigens described herein, and immunogenic portions thereof, have the ability to induce an immunogenic response. More specifically, the antigens have the ability to induce proliferation and/or cytokine production (*i.e*., interferon-γ and/or interleukin-12 production) in T cells, NK cells, B cells and/or macrophages derived from an *M. tuberculosis-immune* individual. The selection of cell type for use in evaluating an immunogenic response to a antigen will depend on the desired response. For example, interleukin-12 production is most readily evaluated using preparations containing B cells and/or macrophages. An *M. tuberculosis-immune* individual is one who is considered to be resistant to the development of tuberculosis by virtue of having mounted an effective T cell response to *M. tuberculosis* (*i.e*., substantially free of disease symptoms). Such individuals may be identified based on a strongly positive (*i.e*., greater than about 10 mm diameter induration) intradermal skin test response to tuberculosis proteins (PPD) and an absence of any signs or symptoms of tuberculosis disease. T cells, NK cells, B cells and macrophages derived from *M. tuberculosis*-immune individuals may be prepared using methods known to those of ordinary skill in the art. For example, a preparation of PBMCs (*i.e*., peripheral blood mononuclear cells) may be employed without further separation of component cells. PBMCs may generally be prepared, for example, using density centrifugation through "FICOLL" (Winthrop Laboratories, NY). T cells for use in the assays described herein may also be purified directly from PBMCs. Alternatively, an enriched T cell line reactive against mycobacterial proteins, or T cell clones reactive to individual mycobacterial proteins, may be employed. Such T cell clones may be generated by, for example, culturing PBMCs from *M. tuberculosis*-immune individuals with mycobacterial proteins for a period of 2-4 weeks. This allows expansion of only the mycobacterial protein-specific T cells, resulting in a line composed solely of such cells. These cells may then be cloned and tested with individual proteins, using methods known to those of ordinary skill in the art, to more accurately define individual T cell specificity. In general, antigens that test positive in assays for proliferation and/or cytokine production (*i.e*., interferon-γ and/or interleukin-12 production) performed using T cells, NK cells, B cells and/or macrophages derived from an *M. tuberculosis*-immune individual are considered immunogenic. Such assays may be performed, for example, using the representative procedures described below. Immunogenic portions of such antigens may be identified using similar assays, and may be present within the polypeptides described herein.

The ability of a polypeptide (*e.g*., an immunogenic antigen, or a portion or other variant thereof) to induce cell proliferation is evaluated by contacting the cells (*e.g*., T cells and/or NK cells) with the polypeptide and measuring the proliferation of the cells. In general, the amount of polypeptide that is sufficient for evaluation of about 10⁵ cells ranges from about 10 ng/mL to about 100 µg/mL and preferably is about 10 µg/mL. The incubation of polypeptide with cells is typically performed at 37°C for about six days. Following incubation with polypeptide, the cells are assayed for a proliferative response, which may be evaluated by methods known to those of ordinary skill in the art, such as exposing cells to a pulse of radiolabeled thymidine and measuring the incorporation of label into cellular DNA. In general, a polypeptide that results in at least a three fold increase in proliferation above background (*i.e*., the proliferation observed for cells cultured without polypeptide) is considered to be able to induce proliferation.

The ability of a polypeptide to stimulate the production of interferon-γ and/or interleukin-12 in cells may be evaluated by contacting the cells with the polypeptide and measuring the level of interferon-γ or interleukin-12 produced by the cells. In general, the amount of polypeptide that is sufficient for the evaluation of about 10⁵ cells ranges from about 10 ng/mL to about 100 µg/mL and preferably is about 10 µg/mL. The polypeptide may be, but need not be, immobilized on a solid support, such as a bead or a biodegradable microsphere, such as those described in U.S. Patent Nos. 4,897,268 and 5,075,109. The incubation of polypeptide with the cells is typically performed at 37°C for about six days. Following incubation with polypeptide, the cells are assayed for interferon-γ and/or interleukin-12 (or one or more subunits thereof), which may be evaluated by methods known to those of ordinary skill in the art, such as an enzyme-linked immunosorbent assay (ELISA) or, in the case of IL-12 P70 subunit, a bioassay such as an assay measuring proliferation of T cells. In general, a polypeptide that results in the production of at least 50 pg of interferon-γ per mL of cultured supernatant (containing 10⁴-10⁵ T cells per mL) is considered able to stimulate the production of interferon-γ. A polypeptide that stimulates the production of at least 10 pg/mL of IL-12 P70 subunit, and/or at least 100 pg/mL of IL-12 P40 subunit, per 10⁵ macrophages or B cells (or per 3 x 10⁵ PBMC) is considered able to stimulate the production of IL-12.

In general, immunogenic antigens are those antigens that stimulate proliferation and/or cytokine production (*i.e*., interferon-γ and/or interleukin-12 production) in T cells, NK cells, B cells and/or macrophages derived from at least about 25% of *M. tuberculosis-*immune individuals. Among these immunogenic antigens, polypeptides having superior therapeutic properties may be distinguished based on the magnitude of the responses in the above assays and based on the percentage of individuals for which a response is observed. In addition, antigens having superior therapeutic properties will not stimulate proliferation and/or cytokine production *in vitro* in cells derived from more than about 25% of individuals who are not *M. tuberculosis*-immune, thereby eliminating responses that are not specifically due to *M. tuberculosis*-responsive cells. Those antigens that induce a response in a high percentage of T cell, NK cell, B cell and/or macrophage preparations from *M. tuberculosis*-immune individuals (with a low incidence of responses in cell preparations from other individuals) have superior therapeutic properties.

Antigens with superior therapeutic properties may also be identified based on their ability to diminish the severity of *M. tuberculosis* infection in experimental animals, when administered as a vaccine. Suitable vaccine preparations for use on experimental animals are described in detail below. Efficacy may be determined based on the ability of the antigen to provide at least about a 50% reduction in bacterial numbers and/or at least about a 40% decrease in mortality following experimental infection. Suitable experimental animals include mice, guinea pigs and primates.

### 5.3. ISOLATION OF CODING SEQUENCES

The present invention also relates to nucleic acid molecules that encode fusion polypeptides of *M. tuberculosis.* In a specific embodiment by way of example in Section 6, *infra*, thirteen *M. tuberculosis* fusion coding sequences were constructed. In accordance with the invention, any nucleotide sequence which encodes the amino acid sequence of the fusion protein can be used to generate recombinant molecules which direct the expression of the coding sequence.

In order to clone full-length coding sequences or homologous variants to generate the fusion polynucleotides, labeled DNA probes designed from any portion of the nucleotide sequences or their complements disclosed herein may be used to screen a genomic or cDNA library made from various strains *of M. tuberculosis* to identify the coding sequence of each individual component. Isolation of coding sequences may also be carried out by the polymerase chain reactions (PCR) using two degenerate oligonucleotide primer pools designed on the basis of the coding sequences disclosed herein.

The invention also relates to isolated or purified polynucleotides complementary to the nucleotide sequences of SEQ ID NOS:1, 3, 5, 7, 9, 11, 13, 15, 17, 19, 21, 23 and 25, and polynucleotides that selectively hybridize to such complementary sequences. In a preferred embodiment, a polynucleotide which hybridizes to the sequence of SEQ ID NOS: 1, 3, 5, 7, 9, 11, 13, 15, 17, 19, 21, 23 and 25 or its complementary sequence under conditions of low stringency and encodes a protein that retains the immunogenicity of the fusion proteins of SEQ ID NOS:2, 4, 6, 8, 10, 12, 14, 16, 18, 20, 22, 24 and 26 is provided. By way of example and not limitation, exemplary conditions of low stringency are as follows (see also Shilo and Weinberg, 1981, Proc. Natl. Acad. Sci. USA 78:6789-6792): Filters containing DNA are pretreated for 6 h at 40°C in a solution containing 35% formamide, 5X SSC, 50 mM Tris-HCl (pH 7.5), 5mM EDTA, 0.1% PVP, 0.1% Ficoll, 1% BSA, and 500 µg/mℓ denatured salmon sperm DNA. Hybridizations are carried out in the same solution with the following modifications: 0.02% PVP, 0.02% Ficoll, 0.2% BSA, 100 µg/mℓ salmon sperm DNA, 10% (wt/vol) dextran sulfate, and 5-20 X 10⁶ cpm ³²P-labeled probe is used. Filters are incubated in hybridization mixture for 18-20 h at 40°C, and then washed for 1.5 h at 55°C in a solution containing 2X SSC, 25 mM Tris-HCl (pH 7.4), 5 mM EDTA, and 0.1% SDS. The wash solution is replaced with fresh solution and incubated an additional 1.5 h at 60°C. Filters are blotted dry and exposed for autoradiography. If necessary, filters are washed for a third time at 65-68°C and re-exposed to film. Other conditions of low stringency which may be used are well known in the art (*e.g*., as employed for cross-species hybridizations).

In another preferred embodiment, a polynucleotide which hybridizes to the coding sequence of SEQ ID NOS:1,3,5,7,9,11,13,15,17,19,21,23 and 25 or its complementary sequence under conditions of high stringency and encodes a protein that retains the immunogenicity of the fusion proteins of SEQ ID NOS:2, 4, 6, 8, 10, 12, 14, 16, 18, 20, 22, 24 and 26 is provided. By way of example and not limitation, exemplary conditions of high stringency are as follows: Prehybridization of filters containing DNA is carried out for 8 h to overnight at 65°C in buffer composed of 6X SSC, 50 mM Tris-HCl (pH 7.5), 1 mM EDTA, 0.02% PVP, 0.02% Ficoll, 0.02% BSA, and 500 µg/mL denatured salmon sperm DNA. Filters are hybridized for 48 h at 65 °C in prehybridization mixture containing 100 µg/mL denatured salmon sperm DNA and 5-20 X 10⁶ cpm of ³²P-labeled probe. Washing of filters is done at 37°C for 1 h in a solution containing 2X SSC, 0.01% PVP, 0.01% Ficoll, and 0.01% BSA. This is followed by a wash in 0.1X SSC at 50°C for 45 min before autoradiography. Other conditions of high stringency which may be used are well known in the art.

In yet another preferred embodiment, a polynucleotide which hybridizes to the coding sequence of SEQ ID NOS:1, 3, 5, 7, 9, 11, 13, 15, 17, 19, 21, 23 and 25 or its complementary sequence under conditions of moderate stringency and encodes a protein that retains the immunogenicity of the fusion proteins of SEQ ID NOS:2, 4, 6, 8, 10, 12, 14, 16, 18, 20, 22, 24 and 26 is provided. Exemplary conditions of moderate stringency are as follows: Filters containing DNA are pretreated for 6 h at 55°C in a solution containing 6X SSC, 5X Denhart's solution, 0.5% SDS and 100 µg/mL denatured salmon sperm DNA. Hybridizations are carried out in the same solution and 5-20 X 10⁶ cpm ³²P-labeled probe is used. Filters are incubated in hybridization mixture for 18-20 h at 55°C, and then washed twice for 30 minutes at 60°C in a solution containing 1X SSC and 0.1% SDS. Filters are blotted dry and exposed for autoradiography. Other conditions of moderate stringency which may be used are well-known in the art. Washing of filters is done at 37°C for 1 h in a solution containing 2X SSC, 0.1% SDS.

### 5.4. POLYPEPTIDES ENCODED BY THE CODING SEQUENCES

In accordance with the invention, a polynucleotide of the invention which encodes a fusion protein, fragments thereof, or functional equivalents thereof may be used to generate recombinant nucleic acid molecules that direct the expression of the fusion protein, fragments thereof, or functional equivalents thereof, in appropriate host cells. The fusion polypeptide products encoded by such polynucleotides may be altered by molecular manipulation of the coding sequence.

Due to the inherent degeneracy of the genetic code, other DNA sequences which encode substantially the same or a functionally equivalent amino acid sequence, may be used in the practice of the invention for the expression of the fusion polypeptides. Such DNA sequences include those which are capable of hybridizing to the coding sequences or their complements disclosed herein under low, moderate or high stringency conditions described in Sections 5.3, *supra.*

Altered nucleotide sequences which may be used in accordance with the invention include deletions, additions or substitutions of different nucleotide residues resulting in a sequence that encodes the same or a functionally equivalent gene product. The gene product itself may contain deletions, additions or substitutions of amino acid residues, which result in a silent change thus producing a functionally equivalent antigenic epitope. Such conservative amino acid substitutions may be made on the basis of similarity in polarity, charge, solubility, hydrophobicity, hydrophilicity, and/or the amphipathic nature of the residues involved. For example, negatively charged amino acids include aspartic acid and glutamic acid; positively charged amino acids include lysine, histidine and arginine; amino acids with uncharged polar head groups having similar hydrophilicity values include the following: glycine, asparagine, glutamine, serine, threonine and tyrosine; and amino acids with nonpolar head groups include alanine, valine, isoleucine, leucine, phenylalanine, proline, methionine and tryptophan.

The nucleotide sequences of the invention may be engineered in order to alter the fusion protein coding sequence for a variety of ends, including but not limited to, alterations which modify processing and expression of the gene product. For example, mutations may be introduced using techniques which are well known in the art, e.g., site-directed mutagenesis, to insert new restriction sites, to alter glycosylation patterns, phosphorylation, etc.

In an alternate embodiment of the invention, the coding sequence of a fusion protein could be synthesized in whole or in part, using chemical methods well known in the art. See, e.g., Caruthers et al., 1980, Nuc. Acids Res. Symp. Ser. 7:215-233; Crea and Horn, 180, Nuc. Acids Res. 9(10):2331; Matteucci and Caruthers, 1980, Tetrahedron Letter 21:719; and Chow and Kempe, 1981, Nuc. Acids Res. 9(12):2807-2817. Alternatively, the polypeptide itself could be produced using chemical methods to synthesize an amino acid sequence in whole or in part. For example, peptides can be synthesized by solid phase techniques, cleaved from the resin, and purified by preparative high performance liquid chromatography. (See Creighton, 1983, Proteins Structures And Molecular Principles, W.H. Freeman and Co., N.Y. pp. 50-60). The composition of the synthetic polypeptides may be confirmed by amino acid analysis or sequencing (*e.g*., the Edman degradation procedure; see Creighton, 1983, Proteins, Structures and Molecular Principles, W.H. Freeman and Co., N.Y., pp. 34-49).

Additionally, the coding sequence of a fusion protein can be mutated *in vitro* or *in vivo*, to create and/or destroy translation, initiation, and/or termination sequences, or to create variations in coding regions and/or form new restriction endonuclease sites or destroy preexisting ones, to facilitate further *in vitro* modification. Any technique for mutagenesis known in the art can be used, including but not limited to, chemical mutagenesis, *in vitro* site-directed mutagenesis (Hutchinson, C., et al., 1978, J. Biol. Chem 253:6551), use of TAB® linkers (Pharmacia), and the like. It is important that the manipulations do not destroy immunogenicity of the fusion polypeptides.

In addition, nonclassical amino acids or chemical amino acid analogs can be introduced as a substitution or addition into the sequence. Non-classical amino acids include, but are not limited to, the D-isomers of the common amino acids, α-amino isobutyric acid, 4-aminobutyric acid, Abu, 2-amino butyric acid, γ-Abu, ∈-Ahx, 6-amino hexanoic acid, Aib, 2-amino isobutyric acid, 3-amino propionic acid, ornithine, norleucine, norvaline, hydroxyproline, sarcosine, citrulline, cysteic acid, t-butylglycine, t-butylalanine, phenylglycine, cyclohexylalanine, β-alanine, fluoro-amino acids, designer amino acids such as β-methyl amino acids, Cα-methyl amino acids, Nα-methyl amino acids, and amino acid analogs in general. Furthermore, the amino acid can be D (dextrorotary) or L (levorotary).

In a specific embodiment, the coding sequences of each antigen in the fusion protein are joined at their amino- or carboxy-terminus via a peptide bond in any order. Alternatively, a peptide linker sequence may be employed to separate the individual polypeptides that make-up a fusion polypeptide by a distance sufficient to ensure that each polypeptide folds into a secondary and tertiary structure that maximizes its antigenic effectiveness for preventing and treating tuberculosis. Such a peptide linker sequence is incorporated into the fusion protein using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes. Preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., Gene 40:39-46, 1985; Murphy et al., Proc. Natl. Acad. Sci. USA 83:8258-8262, 1986; U.S. Patent No. 4,935,233 and U.S. Patent No. 4,751,180. The linker sequence may be from 1 to about 50 amino acids in length. Peptide sequences are not required when the first and second polypeptides have non-essential N-terminal amino acid regions that can be used to separate the functional domains and prevent steric interference. For example, the antigens in a fusion protein may be connected by a flexible polylinker such as Gly-Cys-Gly or Gly-Gly-Gly-Gly-Ser repeated 1 to 3 times (Bird et al., 1988, Science 242:423-426; Chaudhary et al., 1990, Proc. Natl. Acad. Sci. U.S.A. 87:1066-1070).

In one embodiment, such a protein is produced by recombinant expression of a nucleic acid encoding the protein. Such a fusion product can be made by ligating the appropriate nucleic acid sequences encoding the desired amino acid sequences to each other by methods known in the art, in the proper coding frame, and expressing the product by methods known in the art. Alternatively, such a product may be made by protein synthetic techniques, e.g., by use of a peptide synthesizer. Coding sequences for other molecules such as a cytokine or an adjuvant can be added to the fusion polynucleotide as well.

### 5.5. PRODUCTION OF FUSION PROTEINS

In order to produce a *M*. *tuberculosis* fusion protein of the invention, the nucleotide sequence coding for the protein, or a functional equivalent, is inserted into an appropriate expression vector, *i.e*., a vector which contains the necessary elements for the transcription and translation of the inserted coding sequence. The host cells or cell lines transfected or transformed with recombinant expression vectors can be used for a variety of purposes. These include, but are not limited to, large scale production of the fusion protein.

Methods which are well known to those skilled in the art can be used to construct expression vectors containing a fusion coding sequence and appropriate transcriptional/translational control signals. These methods include *in vitro* recombinant DNA techniques, synthetic techniques and *in vivo* recombination/genetic recombination. (*See, e.g*., the techniques described in Sambrook et al., 1989, Molecular Cloning A Laboratory Manual, Cold Spring Harbor Laboratory, N.Y. and Ausubel et al., 1989, Current Protocols in Molecular Biology, Greene Publishing Associates and Wiley Interscience, N.Y.). RNA capable of encoding a polypeptide may also be chemically synthesized (Gait, ed., 1984, Oligonucleoide Synthesis, IRL Press, Oxford).

### 5.5.1. EXPRESSION SYSTEMS

A variety of host-expression vector systems may be utilized to express a fusion protein coding sequence. These include, but are not limited to, microorganisms such as bacteria (*e.g*., *E. coli, B. subtilis*) transformed with recombinant bacteriophage DNA, plasmid DNA or cosmid DNA expression vectors containing a coding sequence; yeast (*e.g*., *Saccharomycdes, Pichia*) transformed with recombinant yeast expression vectors containing a coding sequence; insect cell systems infected with recombinant virus expression vectors (*e.g*., baculovirus) containing a coding sequence; plant cell systems infected with recombinant virus expression vectors (*e.g*., cauliflower mosaic virus, CaMV; tobacco mosaic virus, TMV) or transformed with recombinant plasmid expression vectors (*e.g*., Ti plasmid) containing a coding sequence; or mammalian cell systems (*e.g*., COS, CHO, BHK, 293, 3T3 cells). The expression elements of these systems vary in their strength and specificities.

Depending on the host/vector system utilized, any of a number of suitable transcription and translation elements, including constitutive and inducible promoters, may be used in the expression vector. For example, when cloning in bacterial systems, inducible promoters such as pL of bacteriophage λ, plac, ptrp, ptac (ptrp-lac hybrid promoter; cytomegalovirus promoter) and the like may be used; when cloning in insect cell systems, promoters such as the baculovirus polyhedron promoter may be used; when cloning in plant cell systems, promoters derived from the genome of plant cells (*e.g*., heat shock promoters; the promoter for the small subunit of RUBISCO; the promoter for the chlorophyll α/β binding protein) or from plant viruses (*e.g*., the 35S RNA promoter of CaMV; the coat protein promoter of TMV) may be used; when cloning in mammalian cell systems, promoters derived from the genome of mammalian cells (*e.g*., metallothionein promoter) or from mammalian viruses *(e.g*., the adenovirus late promoter; the vaccinia virus 7.5K promoter) may be used; when generating cell lines that contain multiple copies of a the antigen coding sequence, SV40-, BPV- and EBV-based vectors may be used with an appropriate selectable marker.

Bacterial systems are preferred for the expression *of M. tuberculosis* antigens. For *in vivo* delivery, a bacterium such as *Bacillus-Calmette-Guerrin* may be engineered to express a fusion polypeptide of the invention on its cell surface. A number of other bacterial expression vectors may be advantageously selected depending upon the use intended for the expressed products. For example, when large quantities of the fusion protein are to be produced for formulation of pharmaceutical compositions, vectors which direct the expression of high levels of fusion protein products that are readily purified may be desirable. Such vectors include, but are not limited to, the E. *coli* expression vector pUR278 (Ruther et al., 1983, EMBO J. 2:1791), in which a coding sequence may be ligated into the vector in frame with the *lacZ* coding region so that a hybrid protein is produced; pIN vectors (Inouye and Inouye, 1985, Nucleic Acids Res. 13:3101-3109; Van Heeke and Schuster, 1989, J. Biol. Chem. 264:5503-5509); and the like. pGEX vectors may also be used to express foreign polypeptides as fusion proteins with glutathione S-transferase (GST). In general, such fusion proteins are soluble and can be purified easily from lysed cells by adsorption to glutathione-agarose beads followed by elution in the presence of free glutathione. The pGEX vectors are designed to include thrombin or factor Xa protease cleavage sites so that the cloned fusion polypeptide of interest can be released from the GST moiety.

### 5.5.2. PROTEIN PURIFICATION

Once a recombinant protein is expressed, it can be identified by assays based on the physical or functional properties of the product, including radioactive labeling of the product followed by analysis by gel electrophoresis, radioimmunoassay, ELISA, bioassays, etc.

Once the encoded protein is identified, it may be isolated and purified by standard methods including chromatography (*e.g*., high performance liquid chromatography, ion exchange, affinity, and sizing column chromatography), centrifugation, differential solubility, or by any other standard technique for the purification of proteins. The actual conditions used will depend, in part, on factors such as net charge, hydrophobicity, hydrophilicity, etc., and will be apparent to those having skill in the art. The functional properties may be evaluated using any suitable assay such as antibody binding, induction of T cell proliferation, stimulation of cytokine production such as IL2, IL-4 and IFN-γ. For the practice of the present invention, it is preferred that each fusion protein is at least 80% purified from other proteins. It is more preferred that they are at least 90% purified. For *in vivo* administration, it is preferred that the proteins are greater than 95% purified.

### 5.6. USES OF THE FUSION PROTEIN CODING SEQUENCE

The fusion protein coding sequence of the invention may be used to encode a protein product for use as an immunogen to induce and/or enhance immune responses to *M*. *tuberculosis.* In addition, such coding sequence may be ligated with a coding sequence of another molecule such as cytokine or an adjuvant. Such polynucleotides may be used in *vivo* as a DNA vaccine (U.S. Patent Nos. 5,589,466; 5,679,647; 5,703,055). In this embodiment of the invention, the polynucleotide expresses its encoded protein in a recipient to directly induce an immune response. The polynucleotide may be injected into a naive subject to prime an immune response to its encoded product, or administered to an infected or immunized subject to enhance the secondary immune responses.

In a preferred embodiment, a therapeutic composition comprises a fusion protein coding sequence or fragments thereof that is part of an expression vector. In particular, such a polynucleotide contains a promoter operably linked to the coding region, said promoter being inducible or constitutive, and, optionally, tissue-specific. In another embodiment, a polynucleotide contains a coding sequence flanked by regions that promote homologous recombination at a desired site in the genome, thus providing for intrachromosomal expression of the coding sequence (Koller and Smithies, 1989, Proc. Natl. Acad. Sci. USA 86:8932-8935; Zijlstra et al., 1989, Nature 342:435-438).

Delivery of the nucleic acid into a subject may be either direct, in which case the subject is directly exposed to the nucleic acid or nucleic acid-carrying vector, or indirect, in which case, cells are first transformed with the nucleic acid *in vitro,* then transplanted into the subject. These two approaches are known, respectively, as *in vivo* or *ex vivo* gene transfer.

In a specific embodiment, the nucleic acid is directly administered *in vivo*, where it is expressed to produce the encoded fusion protein product. This can be accomplished by any of numerous methods known in the art, *e.g*., by constructing it as part of an appropriate nucleic acid expression vector and administering it so that it becomes intracellular, *e.g*., by infection using a defective or attenuated retroviral or other viral vector (*see,* U.S. Patent No. 4,980,286), or by direct injection of naked DNA, or by use of microparticle bombardment (e.g., a gene gun; Biolistic, Dupont), or coating with lipids or cell-surface receptors or transfecting agents, encapsulation in liposomes, microparticles, or microcapsules (United States Patent Nos. 5,407,609; 5,853,763; 5,814,344 and 5,820,883), or by administering it in linkage to a peptide which is known to enter the nucleus, by administering it in linkage to a ligand subject to receptor-mediated endocytosis (*see*, *e.g*., Wu and Wu, 1987, J. Biol. Chem. 262:4429-4432) which can be used to target cell types specifically expressing the receptors, etc. In another embodiment, a nucleic acid-ligand complex can be formed in which the ligand comprises a fusogenic viral peptide to disrupt endosomes, allowing the nucleic acid to avoid lysosomal degradation. In yet another embodiment, the nucleic acid can be targeted *in vivo* for cell specific uptake and expression, by targeting a specific receptor (*see*, *e.g*., PCT Publications WO 92/06180 dated April 16, 1992; WO 92/22635 dated December 23, 1992; WO92/20316 dated November 26, 1992; WO93/14188 dated July 22, 1993; WO 93/20221 dated October 14, 1993). Alternatively, the nucleic acid can be introduced intracellularly and incorporated within host cell DNA for expression, by homologous recombination (Koller and Smithies, 1989, Proc. Natl. Acad. Sci. USA 86:8932-8935; Zijlstra et al., 1989, Nature 342:435-438).

In a specific embodiment, a viral vector such as a retroviral vector can be used (*see*, Miller et al., 1993, Meth. Enzymol. 217:581-599). Retroviral vectors have been modified to delete retroviral sequences that are not necessary for packaging of the viral genome and integration into host cell DNA. A fusion coding sequence is cloned into the vector, which facilitates delivery of the nucleic acid into a recipient. More detail about retroviral vectors can be found in Boesen et al., 1994, Biotherapy 6:291-302, which describes the use of a retroviral vector to deliver the *mdr1* gene to hematopoietic stem cells in order to make the stem cells more resistant to chemotherapy. Other references illustrating the use of retroviral vectors in gene therapy are: Clowes et al., 1994, J. Clin. Invest. 93:644-651; Kiem et al., 1994, Blood 83:1467-1473; Salmons and Gunzberg, 1993, Human Gene Therapy 4:129-141; and Grossman and Wilson, 1993, Curr. Opin. in Genetics and Devel. 3:110-114.

Adenoviruses are other viral vectors that can be used in gene therapy. Adenoviruses are especially attractive vehicles for delivering genes to respiratory epithelia. Adenoviruses naturally infect respiratory epithelia where they cause a mild disease. Other targets for adenovirus-based delivery systems are liver, the central nervous system, endothelial cells, and muscle. Adenoviruses have the advantage of being capable of infecting non-dividing cells. Adeno-associated virus (AAV) has also been proposed for use in *in vivo* gene transfer (Walsh et al., 1993, Proc. Soc. Exp. Biol. Med. 204:289-300.

Another approach involves transferring a construct to cells in tissue culture by such methods as electroporation, lipofection, calcium phosphate mediated transfection, or viral infection. Usually, the method of transfer includes the transfer of a selectable marker to the cells. The cells are then placed under selection to isolate those cells that have taken up and are expressing the transferred gene. Those cells are then delivered to a subject.

In this embodiment, the nucleic acid is introduced into a cell prior to administration *in vivo* of the resulting recombinant cell. Such introduction can be carried out by any method known in the art, including but not limited to transfection, electroporation, microinjection, infection with a viral or bacteriophage vector containing the nucleic acid sequences, cell fusion, chromosome-mediated gene transfer, microcell-mediated gene transfer, spheroplast fusion, etc. Numerous techniques are known in the art for the introduction of foreign genes into cells (see *e.g*., Loeffler and Behr, 1993, Meth. Enzymol. 217:599-618; Cohen et al., 1993, Meth. Enzymol. 217:618-644; Cline, 1985, Pharmac. Ther. 29:69-92) and may be used in accordance with the present invention.

The polynucleotides of the invention may also be used in the diagnosis of tuberculosis for detection of polynucleotide sequences specific to *M. tuberculosis* in a patient. Such detection may be accomplished, for example, by isolating polynucleotides from a biological sample obtained from a patient suspected of being infected with the bacteria. Upon isolation of polynucleotides from the biological sample, a labeled polynucleotide of the invention that is complementary to one or more of the polynucleotides, will be allowed to hybridize to polynucleotides in the biological sample using techniques of nucleic acid hybridization known to those of ordinary skill in the art. For example, such hybridization may be carried out in solution or with one hybridization partner on a solid support.

### 5.7. THERAPEUTIC AND PROPHYLACTIC USES OF THE FUSION PROTEIN

Purified or partially purified fusion proteins or fragments thereof may be formulated as a vaccine or therapeutic composition. Such composition may include adjuvants to enhance immune responses. In addition, such proteins may be further suspended in an oil emulsion to cause a slower release of the proteins *in vivo* upon injection. The optimal ratios of each component in the formulation may be determined by techniques well known to those skilled in the art.

Any of a variety of adjuvants may be employed in the vaccines of this invention to enhance the immune response. Most adjuvants contain a substance designed to protect the antigen from rapid catabolism, such as aluminum hydroxide or mineral oil, and a nonspecific stimulator of immune responses, such as lipid A, *Bortadella pertussis* or *Mycobacterium tuberculosis.* Suitable adjuvants are commercially available and include, for example, Freund's Incomplete Adjuvant and Freund's Complete Adjuvant (Difco Laboratories) and Merck Adjuvant 65 (Merck and Company, Inc., Rahway, NJ). Other suitable adjuvants include alum, biodegradable microspheres, monophosphoryl lipid A, quil A, SBAS1c SBAS2 (Ling et al., 1997, Vaccine 15:1562-1567), SBAS7 and Al(OH)₃.

In the vaccines of the present invention, it is preferred that the adjuvant induces an immune response comprising Th1 aspects. Suitable adjuvant systems include, for example, a combination of monophosphoryl lipid A, preferably 3-de-O-acylated monophosphoryl lipid A (3D-MLP) together with an aluminum salt. An enhanced system involves the combination of a monophosphoryl lipid A and a saponin derivative, particularly the combination of 3D-MLP and the saponin QS21 as disclosed in WO 94/00153, or a less reactogenic composition where the QS21 is quenched with cholesterol as disclosed in WO 96/33739. Previous experiments have demonstrated a clear synergistic effect of combinations of 3D-MLP and QS21 in the induction of both humoral and Th1 type cellular immune responses. A particularly potent adjuvant formation involving QS21, 3D-MLP and tocopherol in an oil-in-water emulsion is described in WO 95/17210 and is a preferred formulation.

Formulations containing an antigen of the present invention may be administered to a subject *per se* or in the form of a pharmaceutical or therapeutic composition. Pharmaceutical compositions comprising the proteins may be manufactured by means of conventional mixing, dissolving, granulating, dragee-making, levigating, emulsifying, encapsulating, entrapping or lyophilizing processes. Pharmaceutical compositions may be formulated in conventional manner using one or more physiologically acceptable carriers, diluents, excipients or auxiliaries which facilitate processing of the polypeptides into preparations which can be used pharmaceutically. Proper formulation is dependent upon the route of administration chosen.

For topical administration, the proteins may be formulated as solutions, gels, ointments, creams, suspensions, etc. as are well-known in the art.

Systemic formulations include those designed for administration by injection, *e.g*. subcutaneous, intravenous, intramuscular, intrathecal or intraperitoneal injection, as well as those designed for transdermal, transmucosal, oral or pulmonary administration.

For injection, the proteins may be formulated in aqueous solutions, preferably in physiologically compatible buffers such as Hanks's solution, Ringer's solution, or physiological saline buffer. The solution may contain formulatory agents such as suspending, stabilizing and/or dispersing agents. Alternatively, the proteins may be in powder form for constitution with a suitable vehicle, *e.g*., sterile pyrogen-free water, before use.

For transmucosal administration, penetrants appropriate to the barrier to be permeated are used in the formulation. Such penetrants are generally known in the art.

For oral administration, a composition can be readily formulated by combining the proteins with pharmaceutically acceptable carriers well known in the art. Such carriers enable the proteins to be formulated as tablets, pills, dragees, capsules, liquids, gels, syrups, slurries, suspensions and the like, for oral ingestion by a subject to be treated. For oral solid formulations such as, for example, powders, capsules and tablets, suitable excipients include fillers such as sugars, such as lactose, sucrose, mannitol and sorbitol; cellulose preparations such as maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP); granulating agents; and binding agents. If desired, disintegrating agents may be added, such as the cross-linked polyvinylpyrrolidone, agar, or alginic acid or a salt thereof such as sodium alginate.

If desired, solid dosage forms may be sugar-coated or enteric-coated using standard techniques.

For oral liquid preparations such as, for example, suspensions, elixirs and solutions, suitable carriers, excipients or diluents include water, glycols, oils, alcohols, etc. Additionally, flavoring agents, preservatives, coloring agents and the like may be added.

For buccal administration, the proteins may take the form of tablets, lozenges, etc. formulated in conventional manner.

For administration by inhalation, the proteins for use according to the present invention are conveniently delivered in the form of an aerosol spray from pressurized packs or a nebulizer, with the use of a suitable propellant, *e.g*., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of, *e.g*., gelatin for use in an inhaler or insufflator may be formulated containing a powder mix of the proteins and a suitable powder base such as lactose or starch.

The proteins may also be formulated in rectal or vaginal compositions such as suppositories or retention enemas, *e.g*., containing conventional suppository bases such as cocoa butter or other glycerides.

In addition to the formulations described previously, the proteins may also be formulated as a depot preparation. Such long acting formulations may be administered by implantation (for example subcutaneously or intramuscularly) or by intramuscular injection. Thus, for example, the proteins may be formulated with suitable polymeric or hydrophobic materials (for example as an emulsion in an acceptable oil) or ion exchange resins, or as sparingly soluble derivatives, for example, as a sparingly soluble salt.

Alternatively, other pharmaceutical delivery systems may be employed. Liposomes and emulsions are well known examples of delivery vehicles that may be used to deliver an antigen. Certain organic solvents such as dimethylsulfoxide also may be employed, although usually at the cost of greater toxicity. The fusion proteins may also be encapsulated in microspheres (United States Patent Nos. 5,407,609; 5,853,763; 5,814,344 and 5,820,883). Additionally, the proteins may be delivered using a sustained-release system, such as semipermeable matrices of solid polymers containing the therapeutic or vaccinating agent. Various sustained-release materials have been established and are well known by those skilled in the art. Sustained-release capsules may, depending on their chemical nature, release the proteins for a few weeks up to over 100 days. Depending on the chemical nature and the biological stability of the reagent, additional strategies for protein stabilization may be employed.

Determination of an effective amount of the fusion protein for inducing an immune response in a subject is well within the capabilities of those skilled in the art, especially in light of the detailed disclosure provided herein.

An effective dose can be estimated initially from *in vitro* assays. For example, a dose can be formulated in animal models to achieve an induction of an immune response using techniques that are well known in the art. One having ordinary skill in the art could readily optimize administration to humans based on animal data. Dosage amount and interval may be adjusted individually. For example, when used as a vaccine, the polypeptides and/or polynucleotides of the invention may be administered in about 1 to 3 doses for a 1-36 week period. Preferably, 3 doses are administered, at intervals of about 3-4 months, and booster vaccinations may be given periodically thereafter. Alternate protocols may be appropriate for individual patients. A suitable dose is an amount of polypeptide or DNA that, when administered as described above, is capable of raising an immune response in an immunized patient sufficient to protect the patient from *M. tuberculosis* infection for at least 1-2 years. In general, the amount of polypeptide present in a dose (or produced *in situ* by the DNA in a dose) ranges from about 1 pg to about 100 mg per kg of host, typically from about 10 pg to about 1 mg, and preferably from about 100 pg to about 1 µg. Suitable dose range will vary with the size of the patient, but will typically range from about 0.1 mL to about 5 mL.

### 5.8 DIAGNOSTIC USES OF THE FUSION PROTEIN

The fusion polypeptides of the invention are useful in the diagnosis of tuberculosis infection *in vitro* and *in vivo.* The ability of a polypeptide of the invention to induce cell proliferation or cytokine production can be assayed by the methods disclosed in Section 5.2, *supra.*

In another aspect, this invention provides methods for using one or more of the fusion polypeptides to diagnose tuberculosis using a skin test *in vivo.* As used herein, a skin test is any assay performed directly on a patient in which a delayed-type hypersensitivity (DTH) reaction (such as swelling, reddening or dermatitis) is measured following intradermal injection of one or more polypeptides as described above. Such injection may be achieved using any suitable device sufficient to contact the polypeptide with dermal cells of the patient, such as, for example, a tuberculin syringe or 1 mL syringe. Preferably, the reaction is measured at least about 48 hours after injection, more preferably about 48 to about 72 hours after injection.

The DTH reaction is a cell-mediated immune response, which is greater in patients that have been exposed previously to the test antigen (*i.e*., the immunogenic portion of the polypeptide employed, or a variant thereof). The response may be measured visually, using a ruler. In general, a response that is greater than about 0.5 cm in diameter, preferably greater than about 1.0 cm in diameter, is a positive response, indicative of tuberculosis infection, which may or may not be manifested as an active disease.

The fusion polypeptides of this invention are preferably formulated, for use in a skin test, as pharmaceutical compositions containing a polypeptide and a physiologically acceptable carrier. Such compositions typically contain one or more of the above polypeptides in an amount ranging from about 1 µg to about 100 µg, preferably from about 10 µg to about 50 µg in a volume of 0.1 mL. Preferably, the carrier employed in such pharmaceutical compositions is a saline solution with appropriate preservatives, such as phenol and/or Tween 80™.

In another aspect, the present invention provides methods for using the polypeptides to diagnose tuberculosis. In this aspect, methods are provided for detecting *M. tuberculosis* infection in a biological sample using the fusion polypeptides alone or in combination. As used herein, a "biological sample" is any antibody-containing sample obtained from a patient. Preferably, the sample is whole blood, sputum, serum, plasma, saliva cerebrospinal fluid or urine. More preferably, the sample is a blood, serum or plasma sample obtained from a patient or a blood supply. The polypeptide(s) are used in an assay, as described below, to determine the presence or absence of antibodies to the polypeptide(s) in the sample relative to a predetermined cut-off value. The presence of such antibodies indicates previous sensitization to mycobacterial antigens which may be indicative of tuberculosis.

In embodiments in which more than one fusion polypeptide is employed, the polypeptides used are preferably complementary (*i.e*., one component polypeptide will tend to detect infection in samples where the infection would not be detected by another component polypeptide). Complementary polypeptides may generally be identified by using each polypeptide individually to evaluate serum samples obtained from a series of patients known to be infected with *M. tuberculosis.* After determining which samples test positive (as described below) with each polypeptide, combinations of two or more fusion polypeptides may be formulated that are capable of detecting infection in most, or all, of the samples tested. Such polypeptides are complementary. Approximately 25-30% of sera from tuberculosis-infected individuals are negative for antibodies to any single protein. Complementary polypeptides may, therefore, be used in combination to improve sensitivity of a diagnostic test.

There are a variety of assay formats known to those of ordinary skill in the art for using one or more polypeptides to detect antibodies in a sample. *See, e.g*., Harlow and Lane, Antibodies: A Laboratory Manual, Cold Spring Harbor Laboratory, 1988, which is incorporated herein by reference. In a preferred embodiment, the assay involves the use of polypeptide immobilized on a solid support to bind to and remove the antibody from the sample. The bound antibody may then be detected using a detection reagent that contains a reporter group. Suitable detection reagents include antibodies that bind to the antibody/polypeptide complex and free polypeptide labeled with a reporter group (*e.g*., in a semi-competitive assay). Alternatively, a competitive assay may be utilized, in which an antibody that binds to the polypeptide is labeled with a reporter group and allowed to bind to the immobilized antigen after incubation of the antigen with the sample. The extent to which components of the sample inhibit the binding of the labeled antibody to the polypeptide is indicative of the reactivity of the sample with the immobilized polypeptide.

The solid support may be any solid material known to those of ordinary skill in the art to which the antigen may be attached. For example, the solid support may be a test well in a microtiter plate or a nitrocellulose or other suitable membrane. Alternatively, the support may be a bead or disc, such as glass, fiberglass, latex or a plastic material such as polystyrene or polyvinylchloride. The support may also be a magnetic particle or a fiber optic sensor, such as those disclosed, for example, in U.S. Patent No. 5,359,681.

The polypeptides may be bound to the solid support using a variety of techniques known to those of ordinary skill in the art. In the context of the present invention, the term "bound" refers to both noncovalent association, such as adsorption, and covalent attachment (which may be a direct linkage between the antigen and functional groups on the support or may be a linkage by way of a cross-linking agent). Binding by adsorption to a well in a microtiter plate or to a membrane is preferred. *In* such cases, adsorption may be achieved by contacting the polypeptide, in a suitable buffer, with the solid support for a suitable amount of time. The contact time varies with temperature, but is typically between about 1 hour and 1 day. In general, contacting a well of a plastic microtiter plate (such as polystyrene or polyvinylchloride) with an amount of polypeptide ranging from about 10 ng to about 1 µg, and preferably about 100 ng, is sufficient to bind an adequate amount of antigen.

Covalent attachment of polypeptide to a solid support may generally be achieved by first reacting the support with a bifunctional reagent that will react with both the support and a functional group, such as a hydroxyl or amino group, on the polypeptide. For example, the polypeptide may be bound to supports having an appropriate polymer coating using benzoquinone or by condensation of an aldehyde group on the support with an amine and an active hydrogen on the polypeptide (*see*, *e.g*., Pierce Immunotechnology Catalog and Handbook. 1991, at A12-A13).

In certain embodiments, the assay is an enzyme linked immunosorbent 1 assay (ELISA). This assay may be performed by first contacting a fusion polypeptide antigen that has been immobilized on a solid support, commonly the well of a microtiter plate, with the sample, such that antibodies to the polypeptide within the sample are allowed to bind to the immobilized polypeptide. Unbound sample is then removed from the immobilized polypeptide and a detection reagent capable of binding to the immobilized antibody-polypeptide complex is added. The amount of detection reagent that remains bound to the solid support is then determined using a method appropriate for the specific detection reagent.

More specifically, once the polypeptide is immobilized on the support as described above, the remaining protein binding sites on the support are typically blocked. Any suitable blocking agent known to those of ordinary skill in the art, such as bovine serum albumin or Tween 20™ (Sigma Chemical Co., St. Louis, MO) may be employed. The immobilized polypeptide is then incubated with the sample, and antibody is allowed to bind to the antigen. The sample may be diluted with a suitable diluent, such as phosphate-buffered saline (PBS) prior to incubation. In general, an appropriate contact time is that period of time that is sufficient to detect the presence of antibody within a *M*. *tuberculosis-*infected sample. Preferably, the contact time is sufficient to achieve a level of binding that is at least 95% of that achieved at equilibrium between bound and unbound antibody. Those of ordinary skill in the art will recognize that the time necessary to achieve equilibrium may be readily determined by assaying the level of binding that occurs over a period of time. At room temperature, an incubation time of about 30 minutes is generally sufficient.

Unbound sample may then be removed by washing the solid support with an appropriate buffer, such as PBS containing 0.1 % Tween 20™. Detection reagent may then be added to the solid support. An appropriate detection reagent is any compound that binds to the immobilized antibody-polypeptide complex and that can be detected by any of a variety of means known to those in the art. Preferably, the detection reagent contains a binding agent (for example, Protein A, Protein G, lectin or free antigen) conjugated to a reporter group. Preferred reporter groups include enzymes (such as horseradish peroxidase), substrates, cofactors, inhibitors, dyes, radionuclides, luminescent groups, fluorescent groups, biotin and colloidal particles, such as colloidal gold and selenium. The conjugation of binding agent to reporter group may be achieved using standard methods known to those of ordinary skill in the art. Common binding agents may also be purchased conjugated to a variety of reporter groups from many commercial sources *(e.g*.. Zymed Laboratories, San Francisco, CA, and Pierce, Rockford. IL).

The detection reagent is then incubated with the immobilized antibody-polypeptide complex for an amount of time sufficient to detect the bound antibody. An appropriate amount of time may generally be determined from the manufacturer's instructions or by assaying the level of binding that occurs over a period of time. Unbound detection reagent is then removed and bound detection reagent is detected using the reporter group. The method employed for detecting the reporter group depends upon the nature of the reporter group. For radioactive groups, scintillation counting or autoradiographic methods are generally appropriate. Spectroscopic methods may be used to detect dyes, luminescent groups and fluorescent groups. Biotin may be detected using avidin, coupled to a different reporter group (commonly a radioactive or fluorescent group or an enzyme). Enzyme reporter groups may generally be detected by the addition of substrate (generally for a specific period of time), followed by spectroscopic or other analysis of the reaction products.

To determine the presence or absence of anti *-M. tuberculosis* antibodies in the sample, the signal detected from the reporter group that remains bound to the solid support is generally compared to a signal that corresponds to a predetermined cut-off value. In one preferred embodiment, the cut-off value is the average mean signal obtained when the immobilized antigen is incubated with samples from an uninfected patient. In general, a sample generating a signal that is three standard deviations above the predetermined cut-off value is considered positive for tuberculosis. In an alternate preferred embodiment, the cut-off value is determined using a Receiver Operator Curve. according to the method of Sackett et al., 1985, Clinical Epidemiology: A Basic Science for Clinical Medicine, Little Brown and Co., pp. 106-107. Briefly, in this embodiment, the cut-off value may be determined from a plot of pairs of true positive rates (i.e., sensitivity) and false positive rates (100%-specificity) that correspond to each possible cut-off value for the diagnostic test result. The cut-off value on the plot that is the closest to the upper left-hand corner (*i.e*., the value that encloses the largest area) is the most accurate cut-off value, and a sample generating a signal that is higher than the cut-off value determined by this method may be considered positive. Alternatively, the cut-off value may be shifted to the left along the plot, to minimize the false positive rate, or to the right, to minimize the false negative rate. In general, a sample generating a signal that is higher than the cut-off value determined by this method is considered positive for tuberculosis.

In a related embodiment, the assay is performed in a rapid flow-through or strip test format, wherein the antigen is immobilized on a membrane, such as nitrocellulose. In the flow-through test, antibodies within the sample bind to the immobilized polypeptide as the sample passes through the membrane. A detection reagent *(e.g*., protein A-colloidal gold) then binds to the antibody-polypeptide complex as the solution containing the detection reagent flows through the membrane. The detection of bound detection reagent may then be performed as described above. In the strip test format, one end of the membrane to which polypeptide is bound is immersed in a solution containing the sample. The sample migrates along the membrane through a region containing detection reagent and to the area of immobilized polypeptide. Concentration of detection reagent at the polypeptide indicates the presence of anti- *M. tuberculosis* antibodies in the sample. Typically, the concentration of detection reagent at that site generates a pattern, such as a line, that can be read visually. The absence of such a pattern indicates a negative result. In general, the amount of polypeptide immobilized on the membrane is selected to generate a visually discernible pattern when the biological sample contains a level of antibodies that would be sufficient to generate a positive signal in an ELISA, as discussed above. Preferably, the amount of polypeptide immobilized on the membrane ranges from about 5 ng to about 1 µg, and more preferably from about 50 ng to about 500 ng. Such tests can typically be performed with a very small amount *(e.g*., one drop) of patient serum or blood.

The invention having been described, the following examples are offered by way of illustration and not limitation.

### 6. EXAMPLE: FUSION PROTEINS OF M. TUBERCULOSIS ANTIGENS RETAIN IMMUNOGENICITY OF THE INDIVIDUAL COMPONENTS

### 6.1. MATERIALS AND METHODS

### 6.1.1. CONSTRUCTION OF FUSION PROTEINS

Coding sequences of *M. tuberculosis* antigens were modified by PCR in order to facilitate their fusion and subsequent expression of fusion protein. DNA amplification was performed using 10 µl 10X Pfu buffer, 2 µl 10 mM dNTPs, 2 µl each of the PCR primers at 10 µM concentration, 81.5 µl water, 1.5 µl Pfu DNA polymerase (Stratagene, La Jolla, CA) and 1 µl DNA at either 70 ng/µl (for TbRa3 antigen) or 50 ng/µl (for 38 kD and Tb38-1 antigens). For TbRa3 antigen, denaturation at 94°C was performed for 2 min, followed by 40 cycles of 96°C for 15 sec and 72°C for 1 min, and lastly by 72°C for 4 min. For 38 kD antigen, denaturation at 96°C was performed for 2 min, followed by 40 cycles of 96°C for 30 sec, 68°C for 15 sec and 72°C for 3 min, and finally by 72°C for 4 min. For Tb38-1 antigen, denaturation at 94°C for 2 min was followed by 10 cycles of 96°C for 15 sec, 68°C for 15 sec and 72°C for 1.5 min, 30 cycles of 96°C for 15 sec, 64°C for 15 sec and 72°C for 1.5, and finally by 72°C for 4 min.

Following digestion with a restriction endonuclease to yield the desired cohesive or blunt ends, a polynucleotide specific for each fusion polypeptide was ligated into an expression plasmid. Each resulting plasmid contained the coding sequences of the individual antigens of each fusion polypeptide. The expression vectors used were pET-12b and pT7^L2 IL 1.

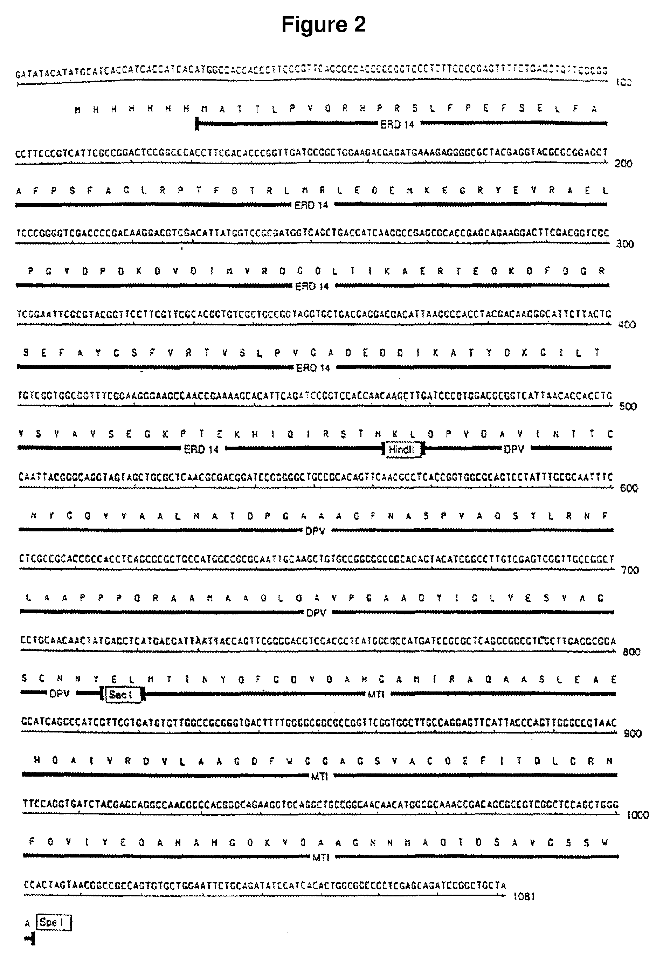

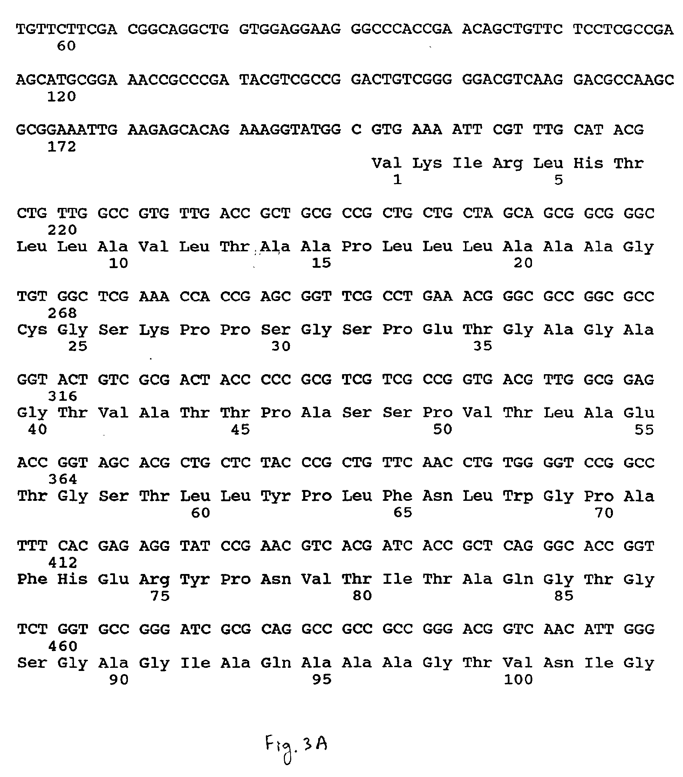

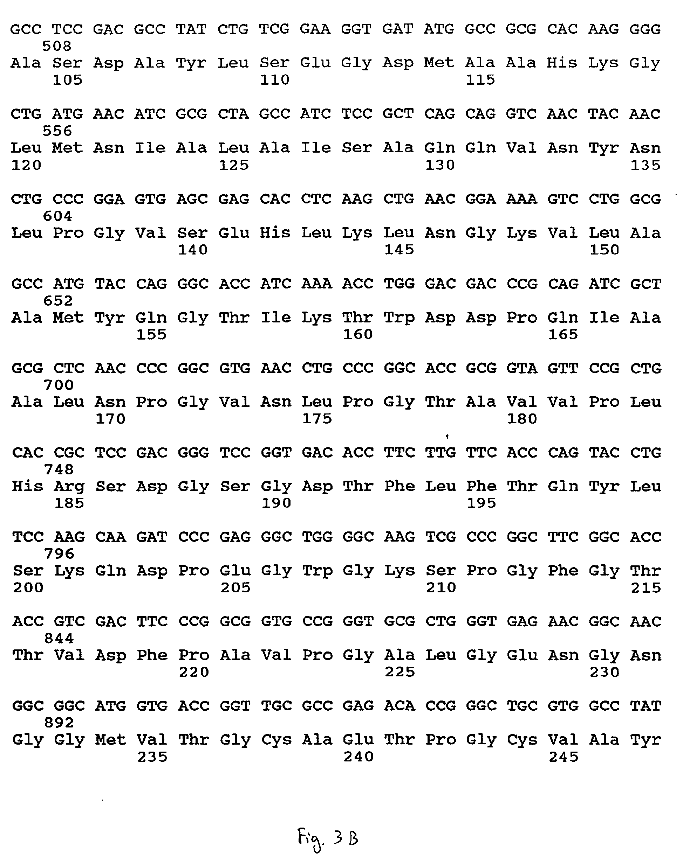

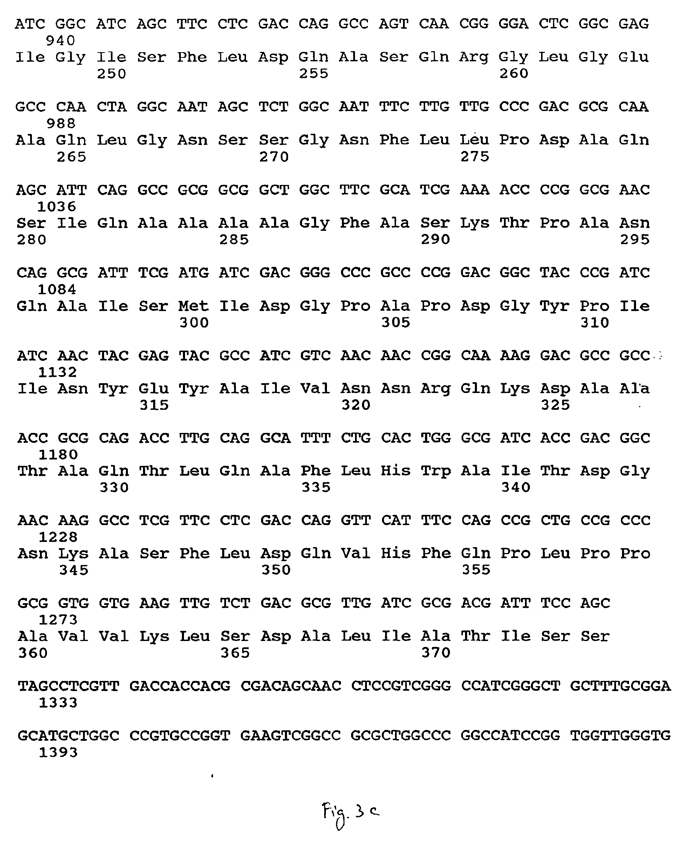

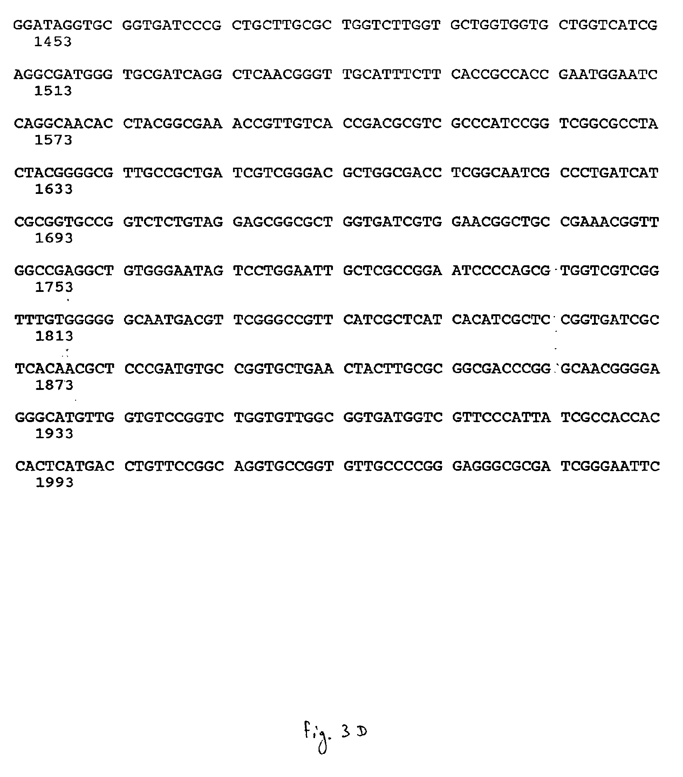

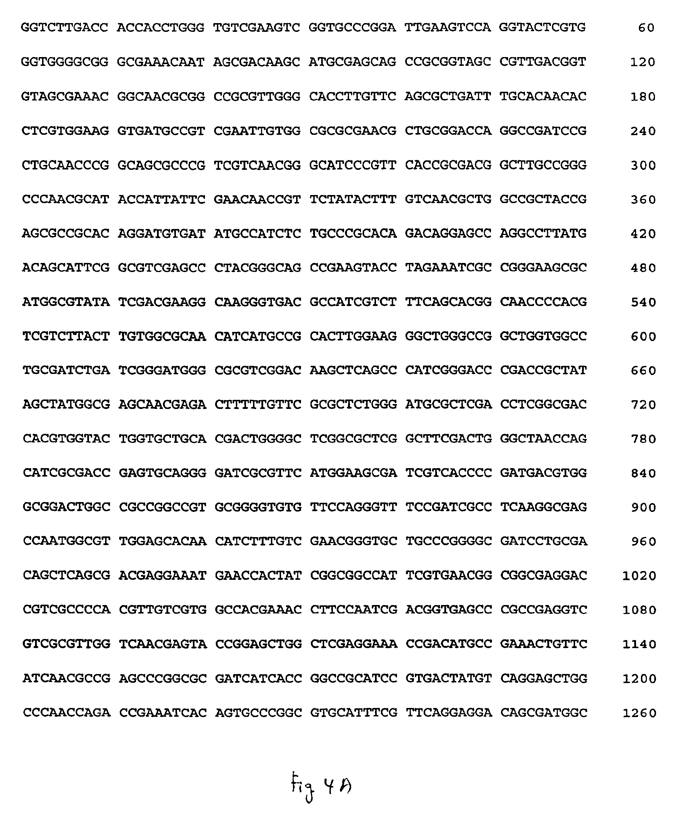

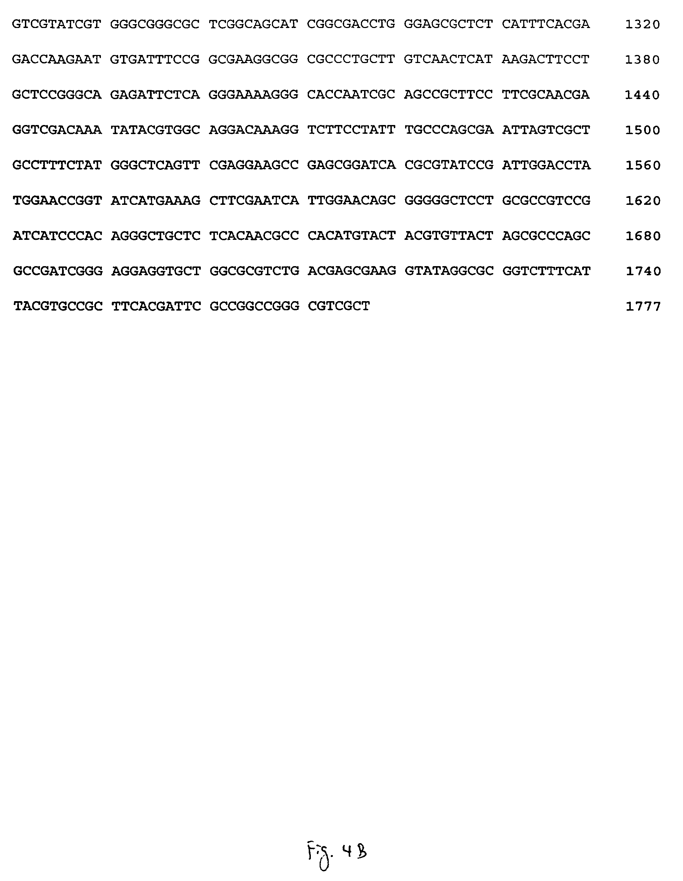

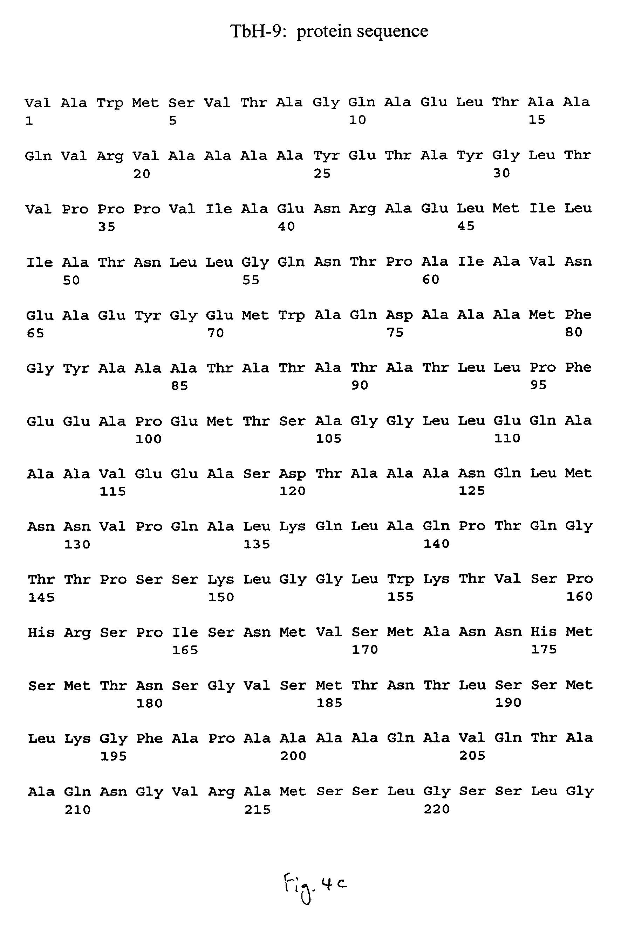

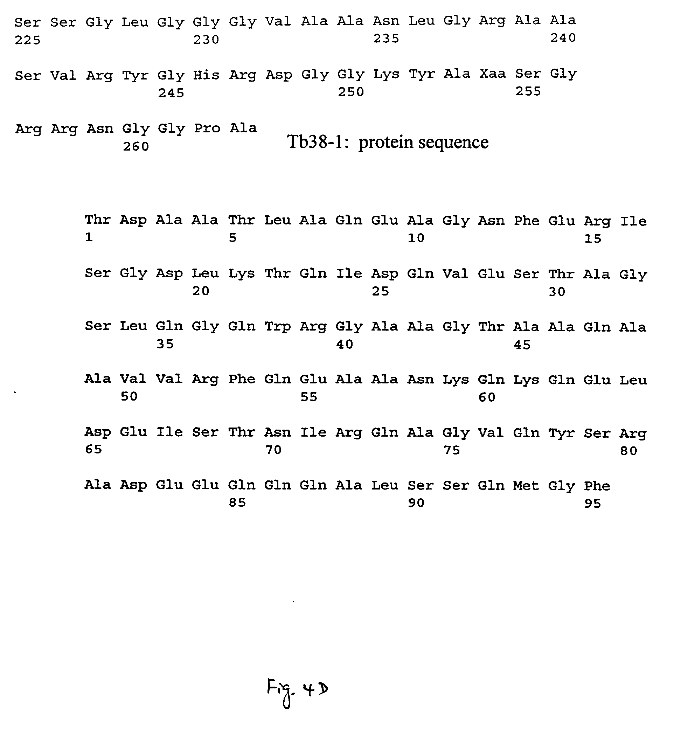

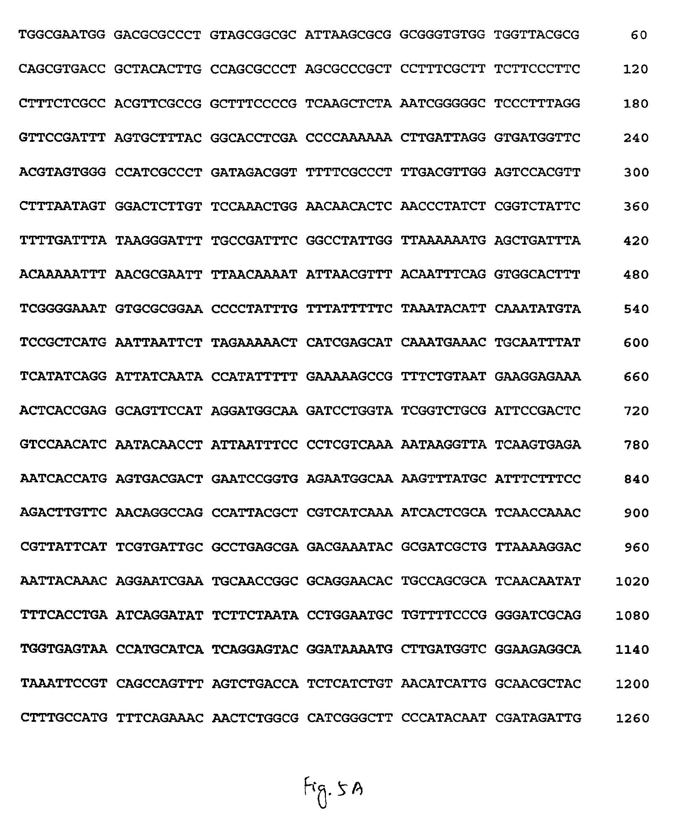

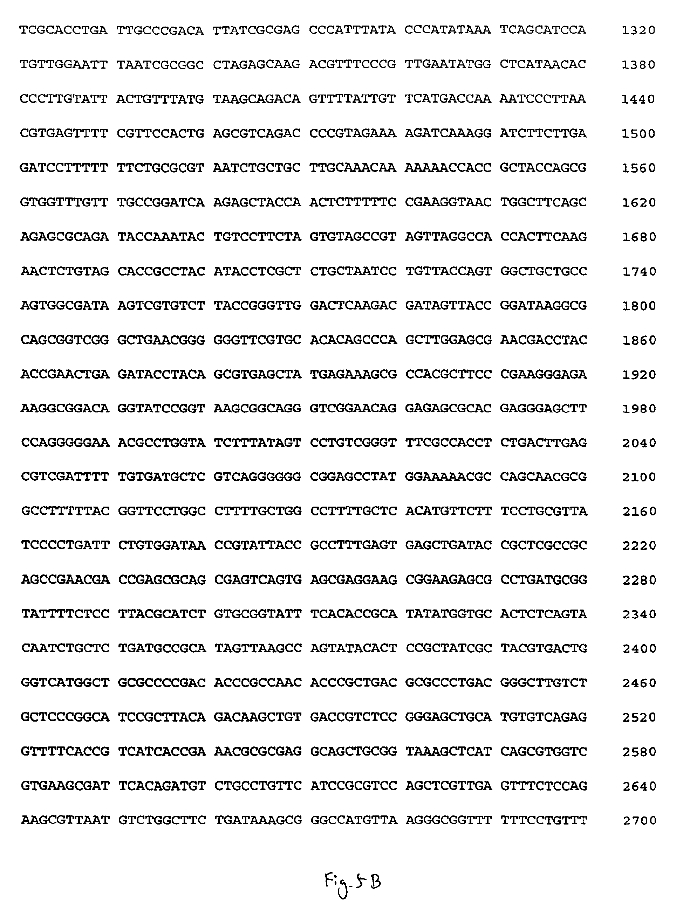

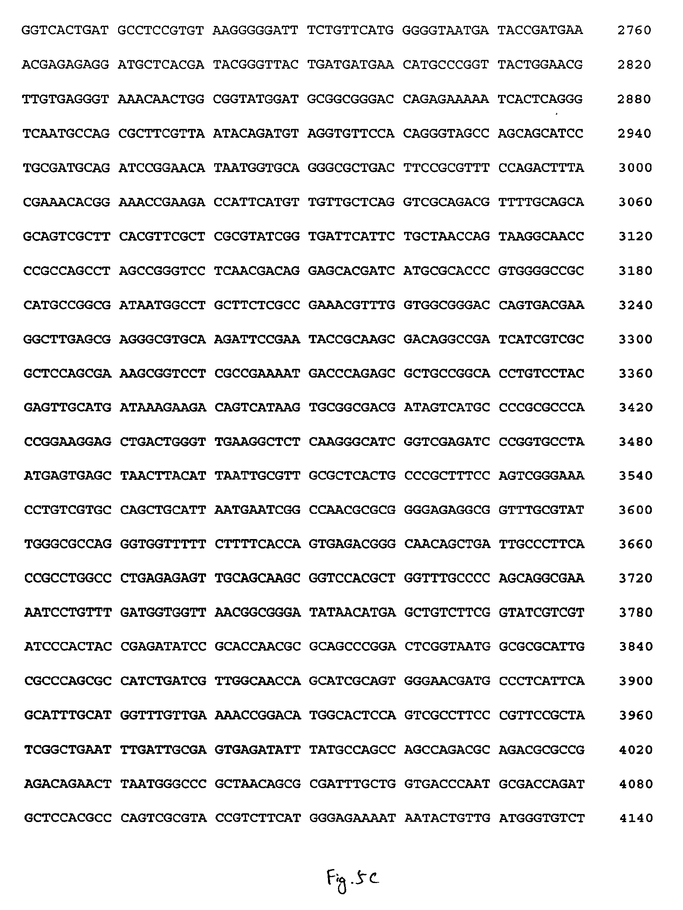

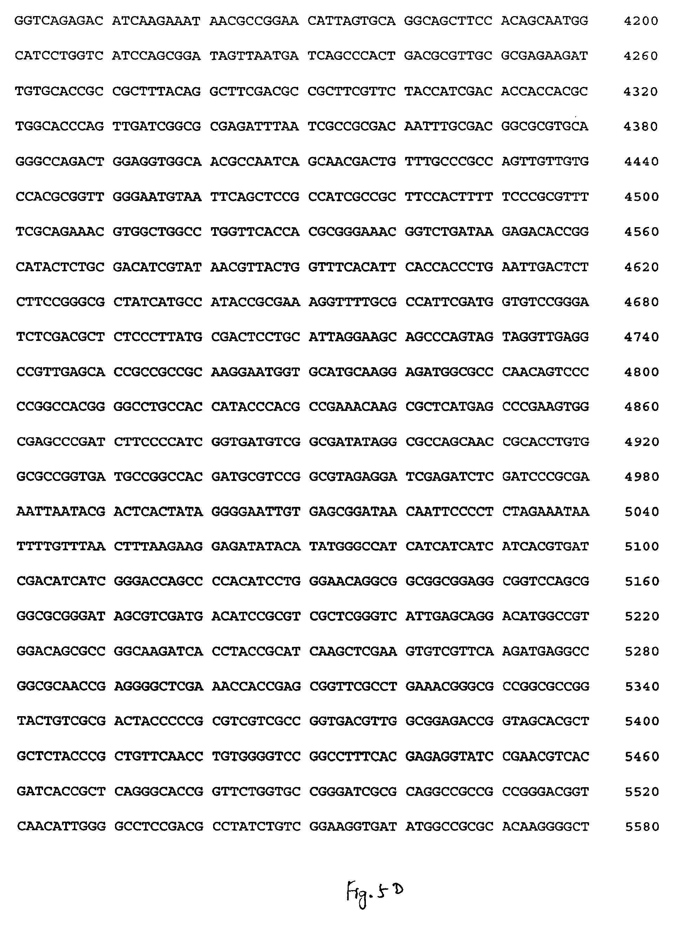

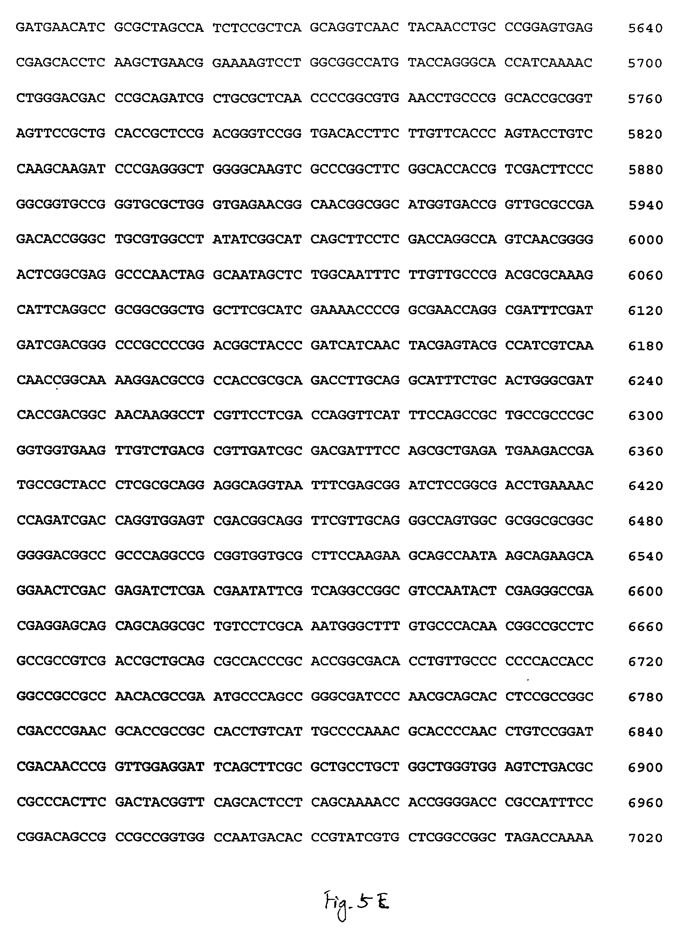

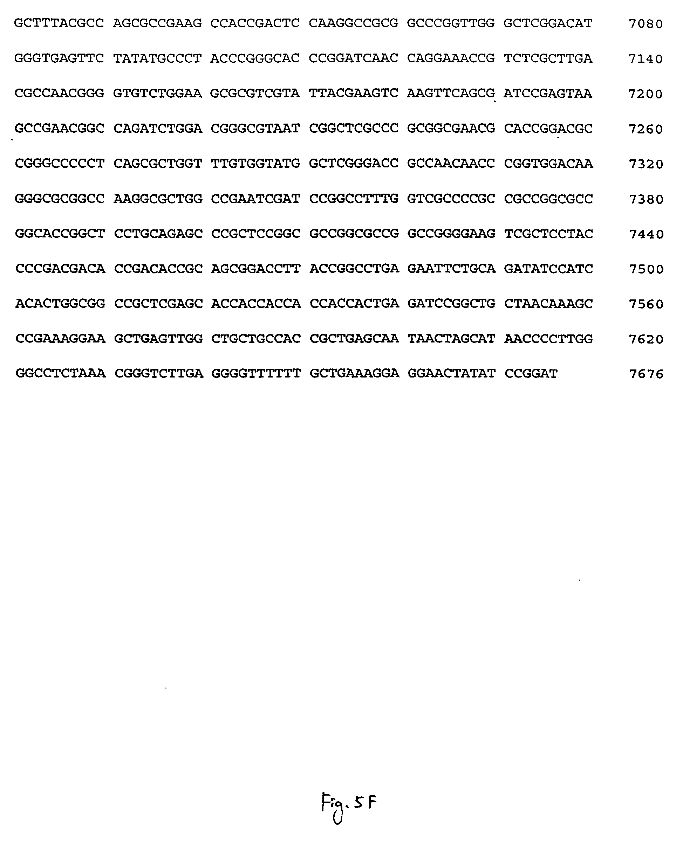

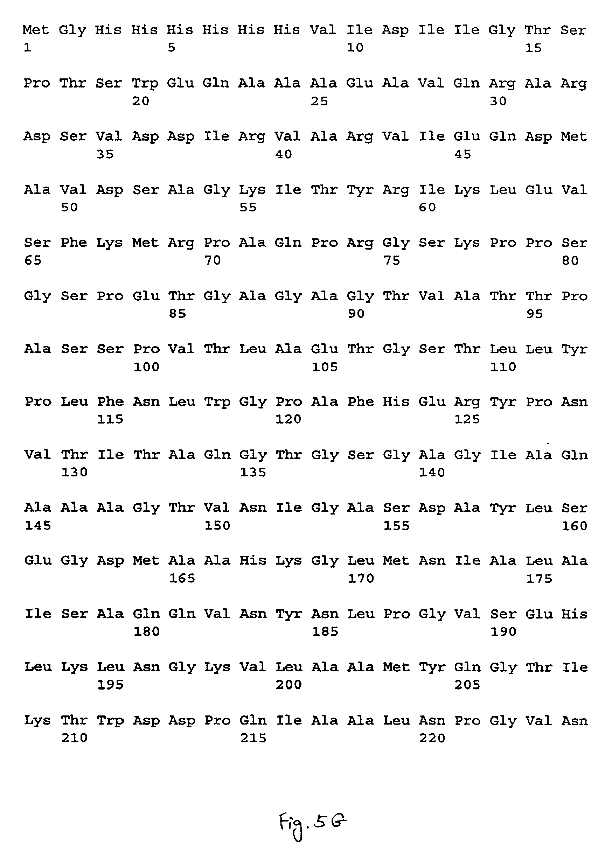

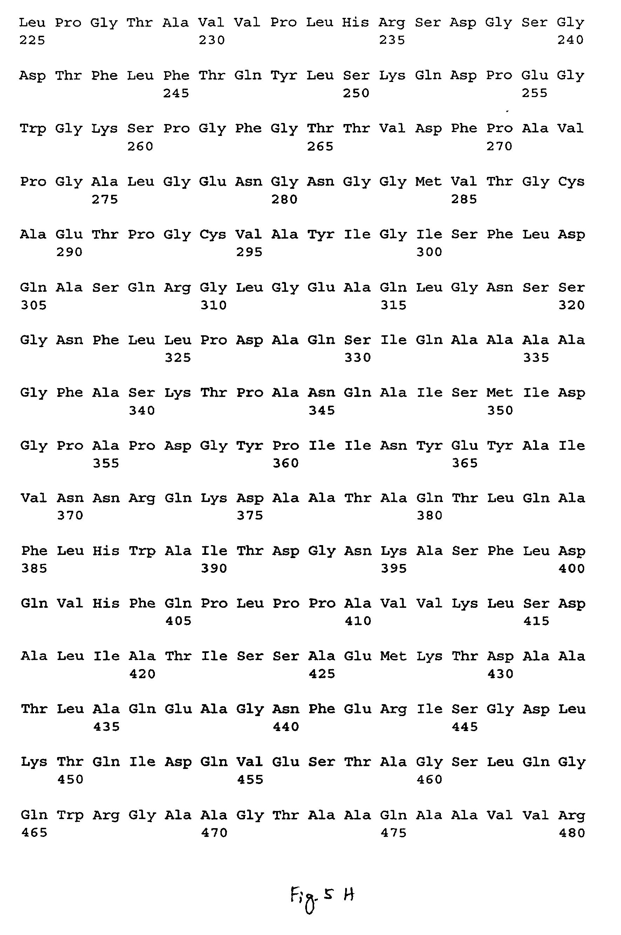

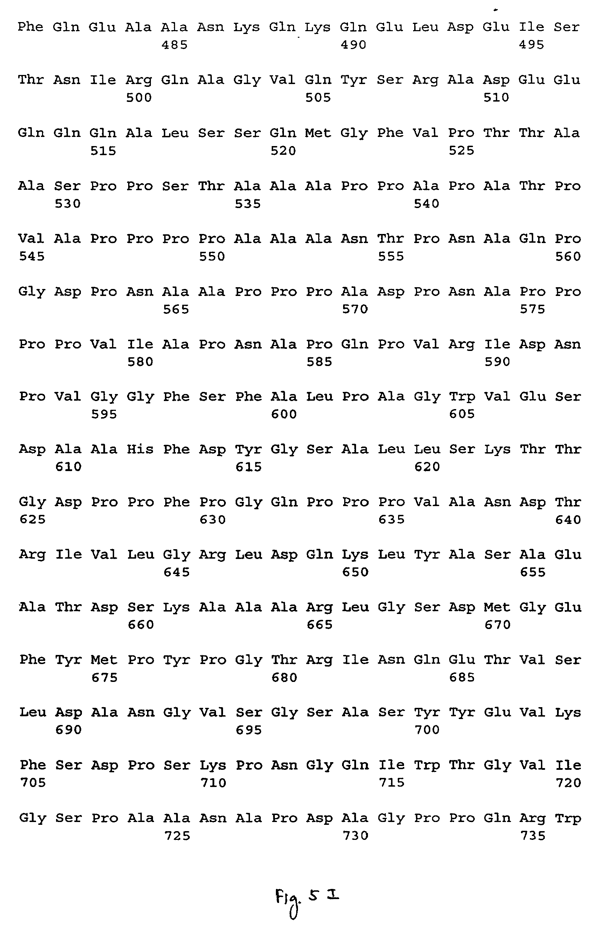

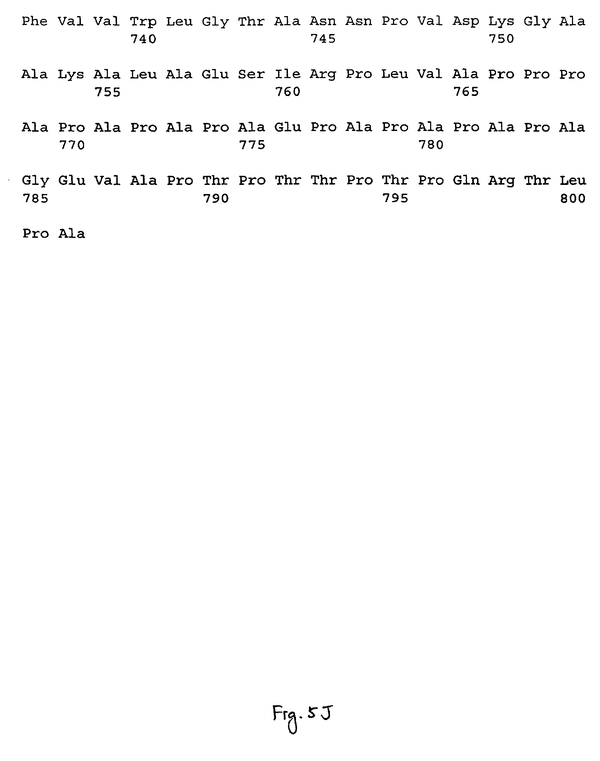

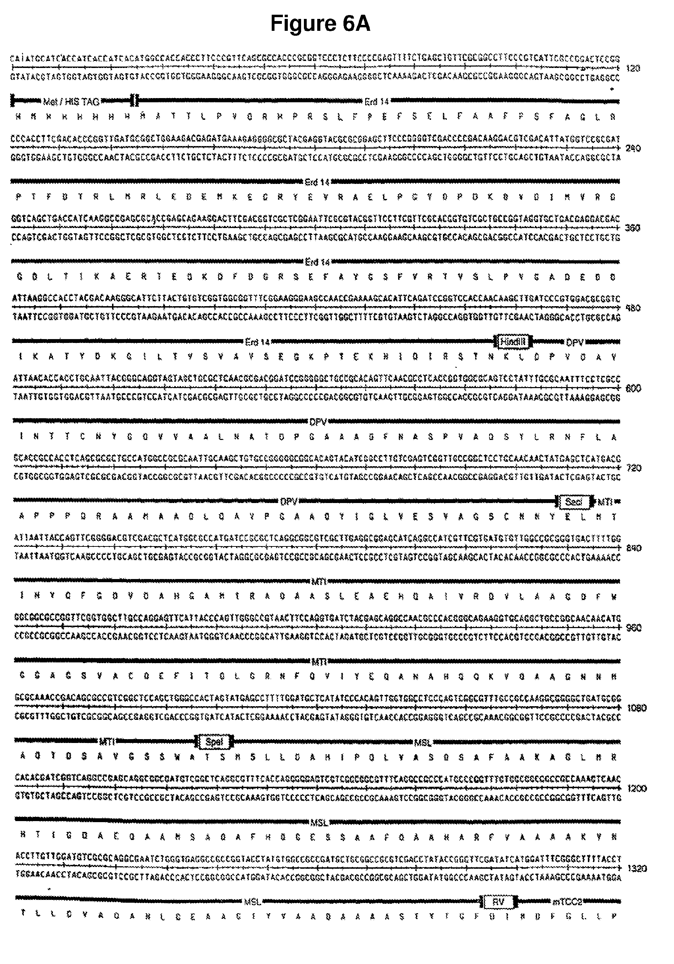

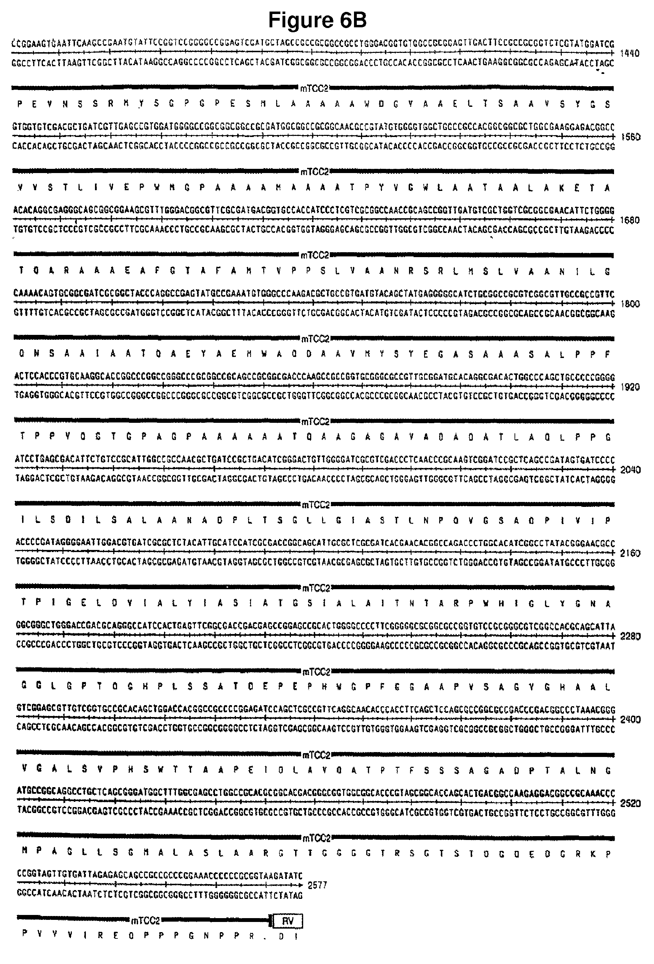

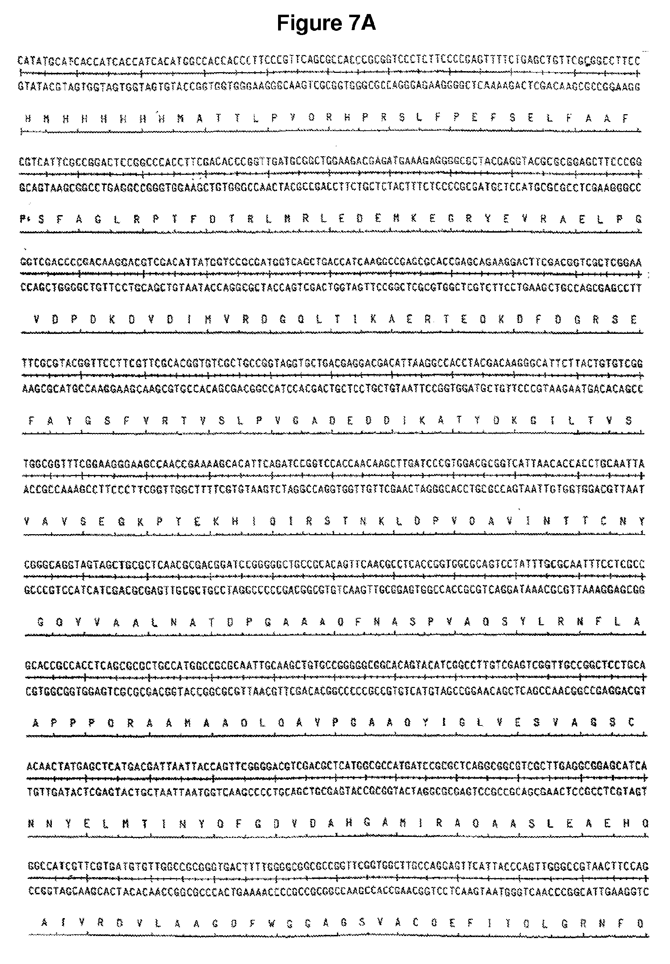

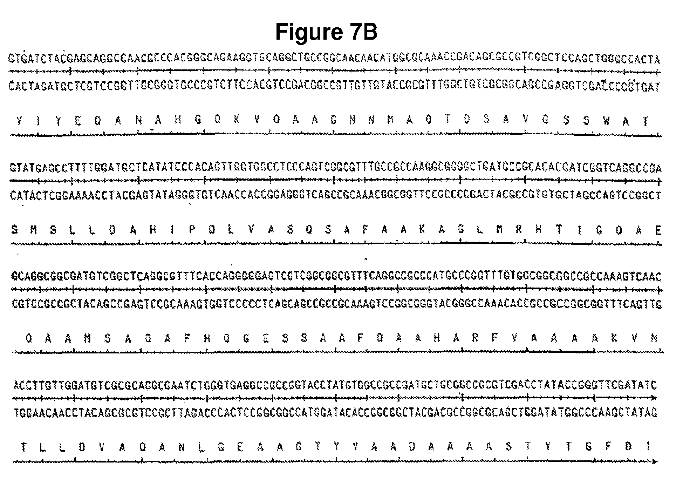

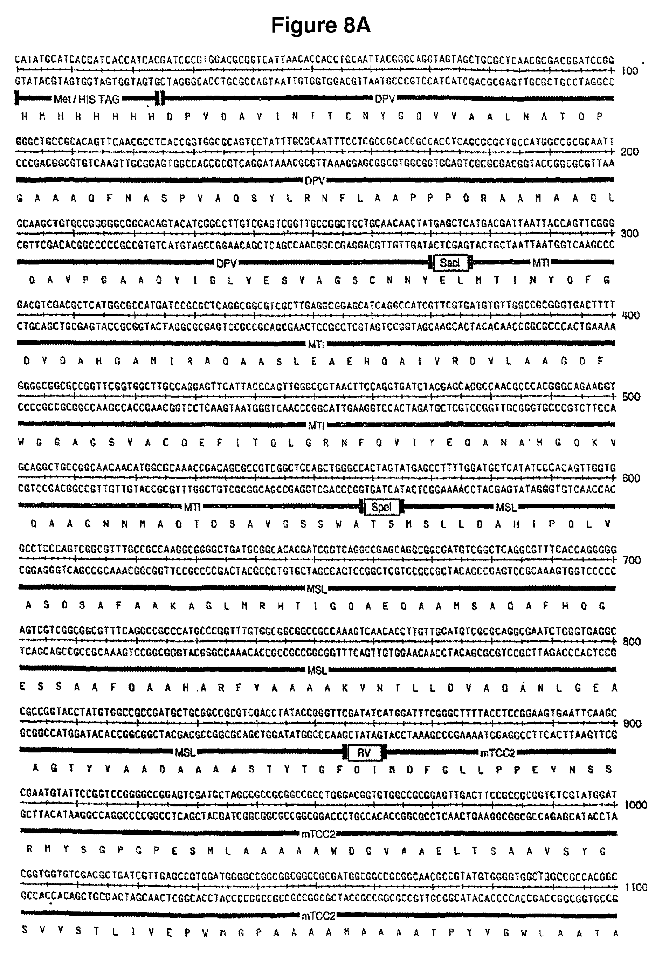

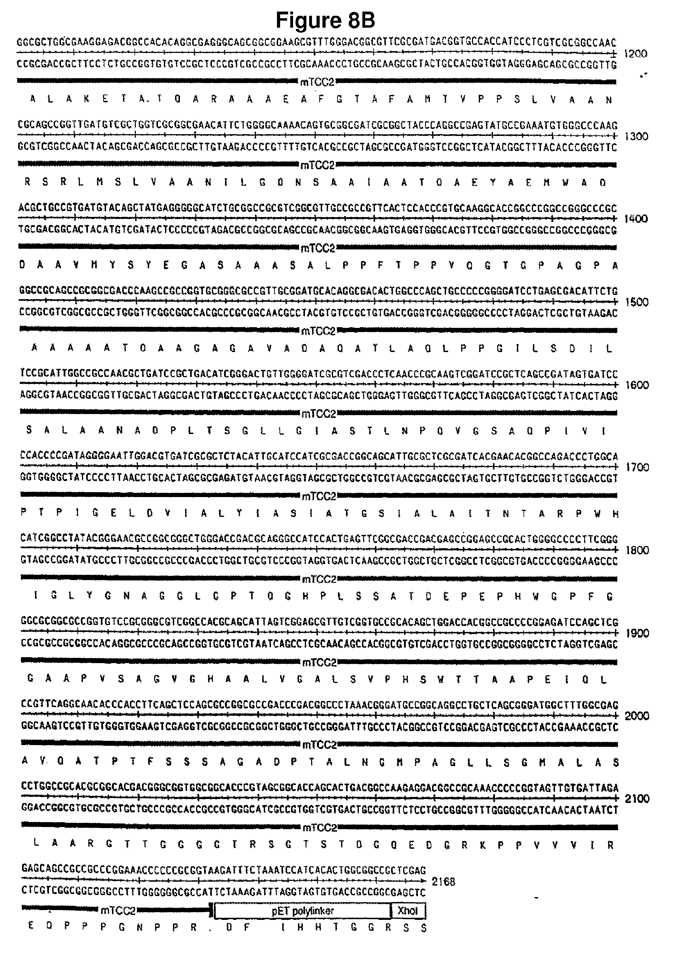

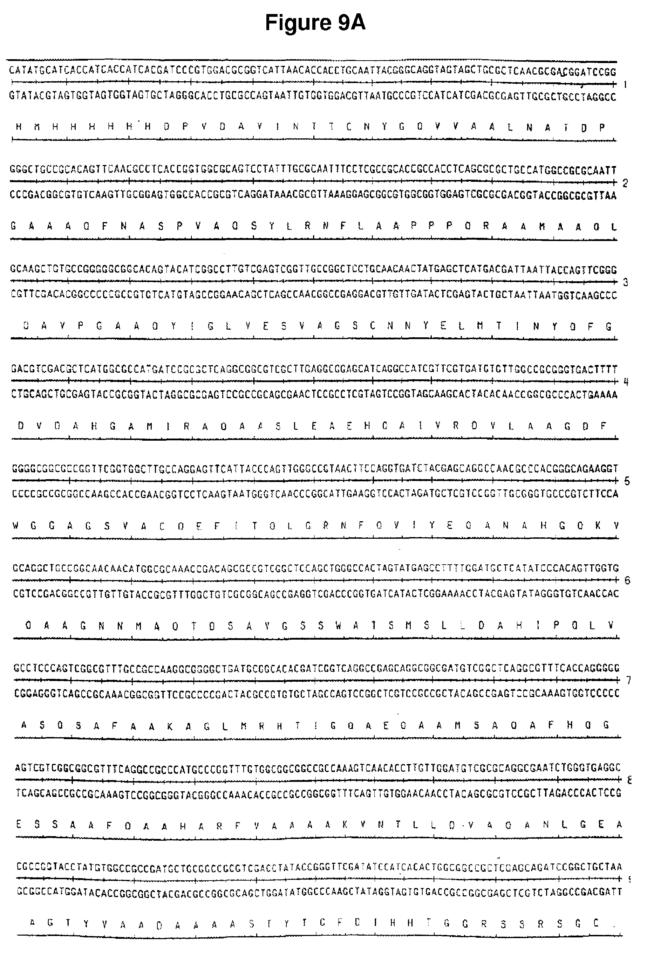

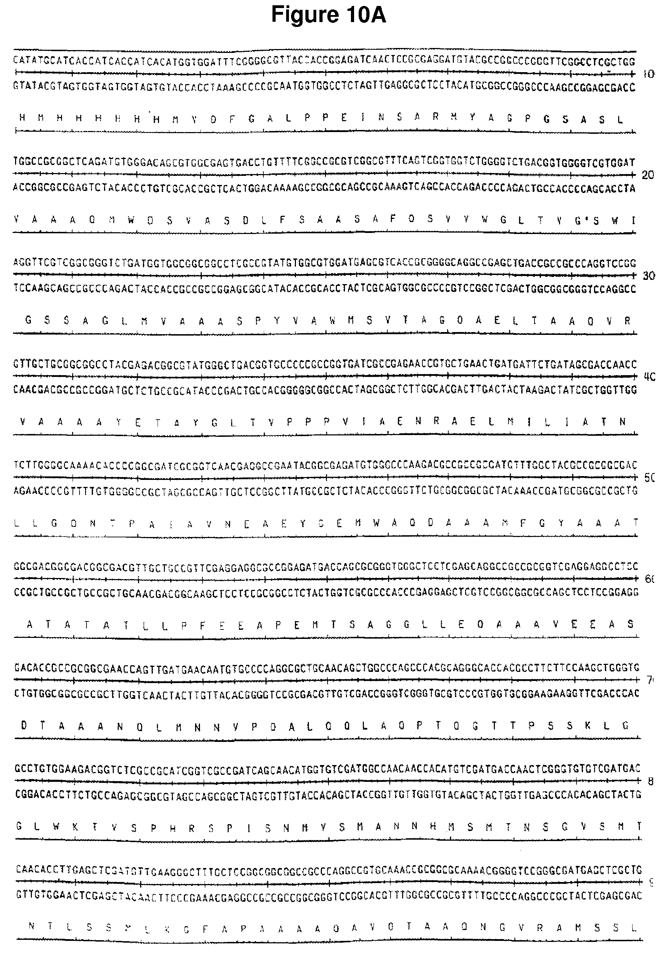

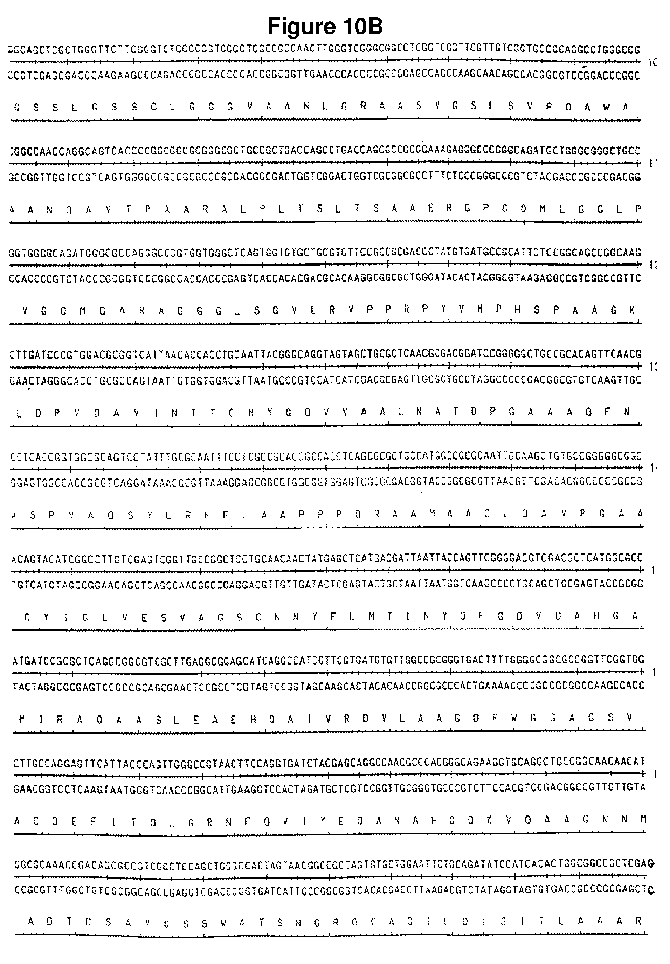

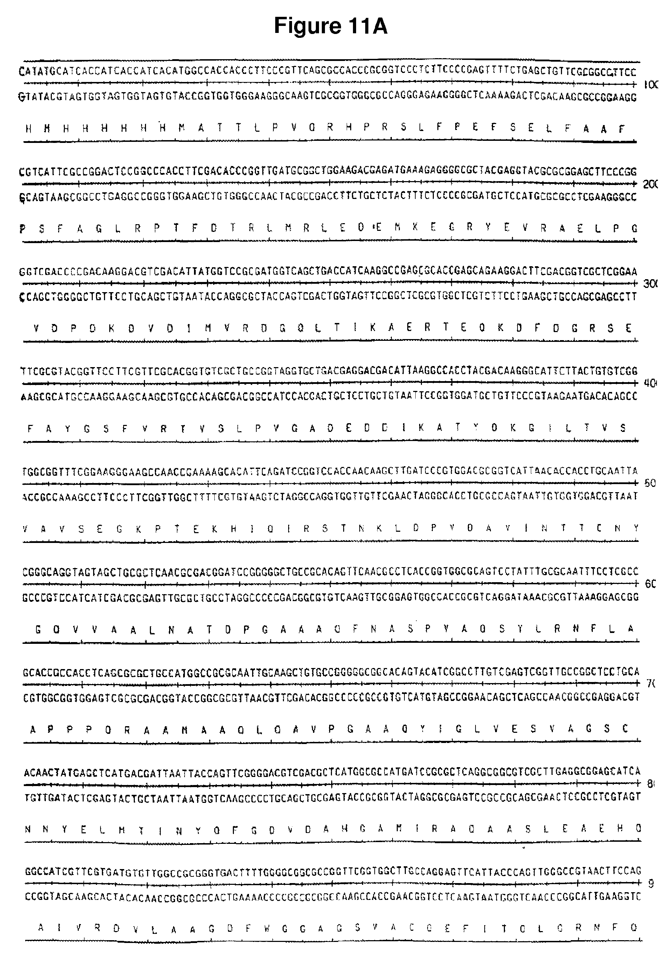

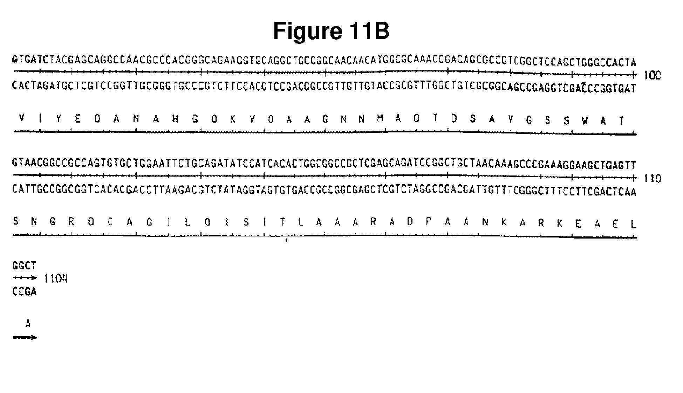

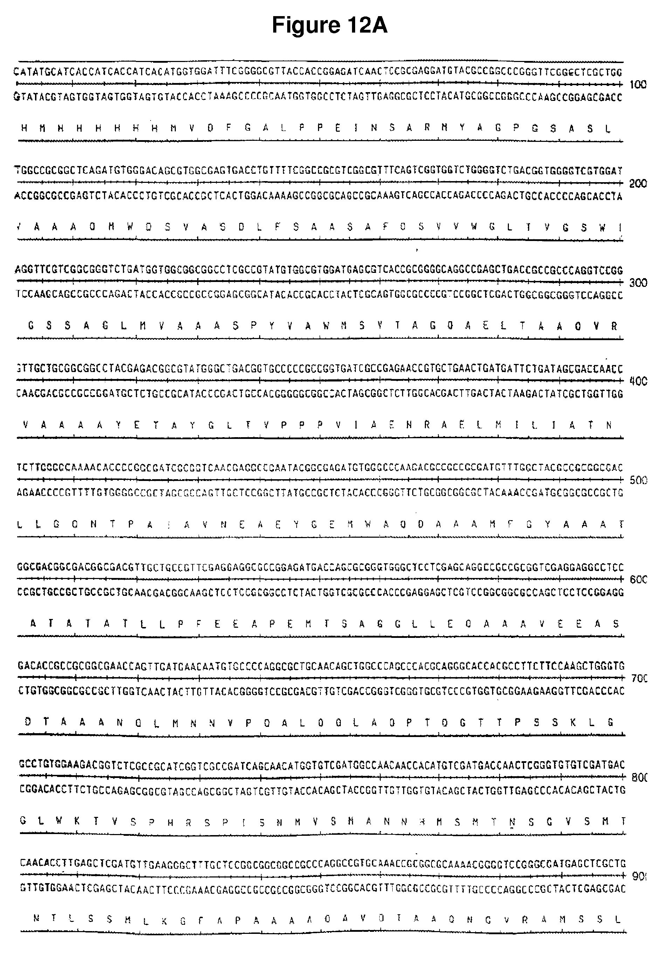

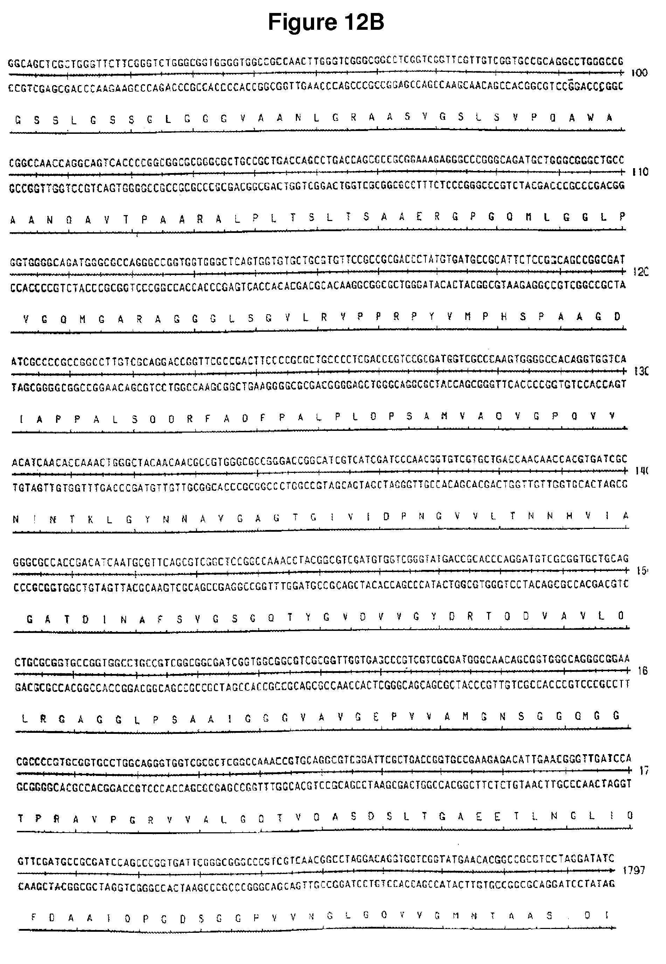

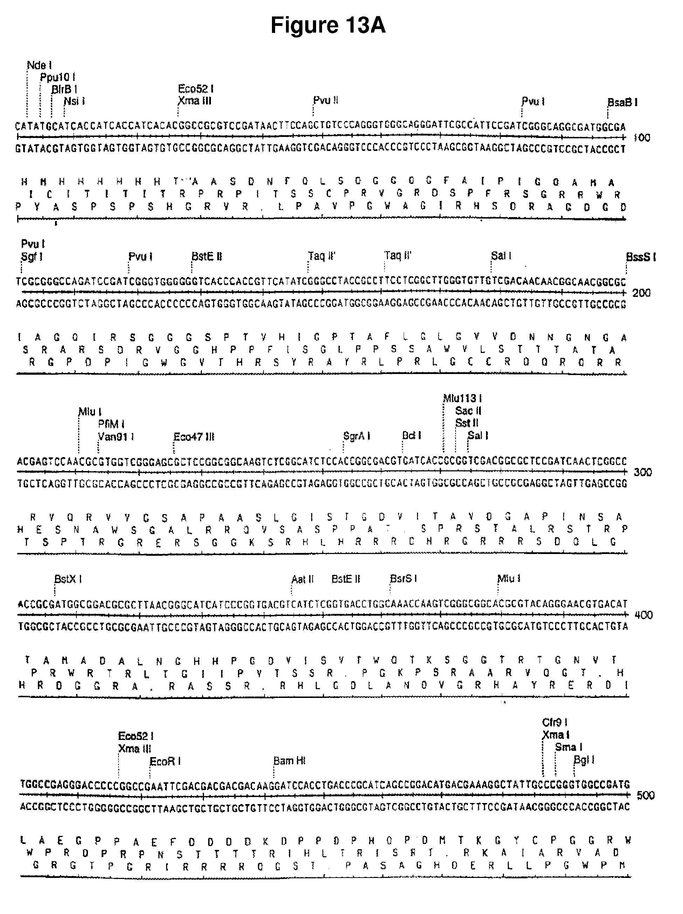

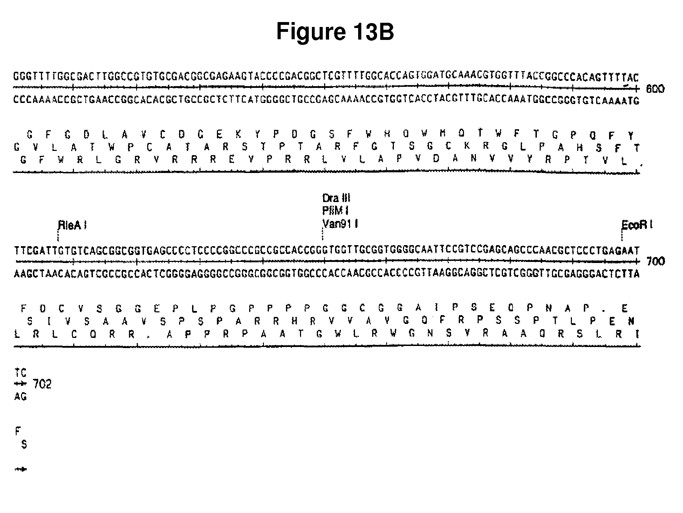

Three coding sequences for antigens Ra12, TbH9 and Ra35 were ligated to encode one fusion protein (SEQ ID NOS:1 and 2) (Fig. 1A and 2B). Another three coding sequences for antigens Erd14, DPV and MTI were ligated to encode a second fusion protein (SEQ ID NOS:3 and **4**) (Fig. 2). Three coding sequences for antigens TbRa3, 38kD and Tb38-1 were ligated to encode one fusion protein (SEQ ID NOS:5 and 6) (Fig. 3A - 3D). Two coding sequences for antigens TbH9 and Tb38-1 were ligated to encode one fusion protein (SEQ ID NOS:7 and 8) (Fig. **4**A - **4**D). Four coding sequences for antigens TbRa3, 38kD, Tb38-1 and DPEP were ligated to encode one fusion protein (SEQ ID NOS:9 and 10) (Fig. 5A - 5J). Five coding sequences for antigens Erd14, DPV, MTI, MSL and MTCC2 were ligated to encode one fusion protein (SEQ ID NOS:11 and 12) (Fig. 6A and 6B). Four coding sequences for antigens Erd14, DPV, MTI and MSL were ligated to encode one fusion protein (SEQ ID NOS:13 and 14) (Fig. 7A and 7B). Four coding sequences for antigens DPV, MTI, MSL and MTCC2 were ligated to encode one fusion protein (SEQ ID NOS:15 and 16) (Fig. 8A and 8B). Three coding sequences for antigens DPV, MTI and MSL were ligated to encode one fusion protein (SEQ ID NOS:17 and 18) (Fig. 9A and 9B). Three coding sequences for antigens TbH9, DPV and MTI were ligated to encode one fusion protein (SEQ ID NOS:19 and 20) (Fig. 10A and 10B). Three coding sequences for antigens Erd14, DPV and MTI were ligated to encode one fusion protein (SEQ ID NOS:21 and 22) (Fig. 11A and 11B). Two coding sequences for antigens TbH9 and Ra35 were ligated to encode one fusion protein (SEQ ID NOS:23 and 24) (Fig. 12A and 12B). Two coding sequences for antigens Ra12 and DPPD were ligated to encode one fusion protein (SEQ ID NOS:25 and 26) (Fig. 13A and 13B).

The recombinant proteins were expressed in *E*. *coli* with six histidine residues at the amino-terminal portion using the pET plasmid vector (pET-17b) and a T7 RNA polymerase expression system (Novagen, Madison, WI). *E*. *coli* strain BL21 (DE3) pLysE (Novagen) was used for high level expression. The recombinant (His-Tag) fusion proteins were purified from the soluble supernatant or the insoluble inclusion body of 500 ml of IPTG induced batch cultures by affinity chromatography using the one step QIAexpress Ni-NTA Agarose matrix (QIAGEN, Chatsworth, CA) in the presence of 8M urea. Briefly, 20 ml of an overnight saturated culture of BL21 containing the pET construct was added into 500 ml of 2xYT media containing 50 µg/ml ampicillin and 34 µg/ml chloramphenicol, grown at 37°C with shaking. The bacterial cultures were induced with 2mM IPTG at an OD *560* of 0.3 and grown for an additional 3 h (OD =1.3 to 1.9). Cells were harvested from 500 ml batch cultures by centrifugation and resuspended in 20 ml of binding buffer (0.1 M sodium phosphate, pH 8.0; 10 mM Tris-HCl, pH 8.0) containing 2mM PMSF and 20 µg/ml leupeptin plus one complete protease inhibitor tablet (Boehringer Mannheim) per 25 ml. *E*. *coli* was lysed by freeze-thaw followed by brief sonication, then spun at 12 k rpm for 30 min to pellet the inclusion bodies.

The inclusion bodies were washed three times in 1% CHAPS in 10 mM Tris-HCl (pH 8.0). This step greatly reduced the level of contaminating LPS. The inclusion body was finally solubilized in 20 ml of binding buffer containing 8 M urea or 8M urea was added directly into the soluble supernatant. Recombinant fusion proteins with His-Tag residues were batch bound to Ni-NTA agarose resin (5 ml resin per 500 ml inductions) by rocking at room temperature for 1 h and the complex passed over a column. The flow through was passed twice over the same column and the column washed three times with 30 ml each of wash buffer (0.1 M sodium phosphate and 10 mM Tris-HCL, pH 6.3) also containing 8 M urea. Bound protein was eluted with 30 ml of 150 mM immidazole in wash buffer and 5 ml fractions collected. Fractions containing each recombinant fusion protein were pooled, dialyzed against 10 mM TrisHCl (pH 8.0) bound one more time to the Ni-NTA matrix, eluted and dialyzed in 10 mM Tris-HCL (pH 7.8). The yield of recombinant protein varies from 25-150 mg per liter of induced bacterial culture with greater than 98% purity. Recombinant proteins were assayed for endotoxin contamination using the *Limulus* assay (BioWhittaker) and were shown to contain < 10 E.U.Img.

### 6.1.2. T-CELL PROLIFERATION ASSAY

Purified fusion polypeptides were tested for the ability to induce T-cell proliferation in peripheral blood mononuclear cell (PBMC) preparations. The PBMCs from donors known to be PPD skin test positive and whose T-cells were shown to proliferate in response to PPD and crude soluble proteins from *M. tuberculosis* were cultured in RPMI 1640 supplemented with 10% pooled human serum and 50 µg/ml gentamicin. Purified polypeptides were added in duplicate at concentrations of 0.5 to 10 µg/ml. After six days of culture in 96-well round-bottom plates in a volume of 200 µl, 50 µl of medium was removed from each well for determination of IFN-γ levels, as described below in Section 6.1.3. The plates were then pulsed with 1 µCi/well of tritiated thymidine for a further 18 hours, harvested and tritium uptake determined using a gas scintillation counter. Fractions that resulted in proliferation in both replicates three fold greater than the proliferation observed in cells cultured in medium alone were considered positive.

### 6.1.3. INTERFERON-γ ASSAY

Spleens from mice were removed asceptically and single cell suspension prepared in complete RPMI following lysis of red blood cells. 100 µl of cells (2x10⁻⁵ cells) were plated per well in a 96-well flat bottom microtiter plate. Cultures were stimulated with the indicated recombinant proteins for 24h and the supernatant assayed for IFN-γ.

The levels of supernatant IFN-γ was analysed by sandwich ELISA, using antibody pairs and procedures available from PharMingen. Standard curves were generated using recombinant mouse cytokines. ELISA plates (Corning) were coated with 50 µl/well (1 µg/ml, in 0.1 M bicarbonate coating buffer, pH9.6) of a cytokine capture mAb (rat antimouse IFN-γ (PharMingen; Cat. # 18181D)), and incubated for 4 h at room temp. Shake out plate contents and block with PBS-0.05% Tween, 1.0% BSA (200 µl/well) overnight at 4°C and washed for 6X in PBS-0.1% Tween. Standards (mouse IFN-γ) and supernatant samples diluted in PBS-0.05% Tween, 0.1% BSA were then added for 2 hr at room temp. The plates were washed as above and then incubated for 2 hr at room temperature with 100 µl/well of a second Ab (biotin rat α mouse IFN-γ (Cat. # 18112D; PharMingen) at 0.5 µg/ml diluted in PBS-0.05% Tween, 0.1 % BSA. After washing, plates were incubated with 100 µl/well of streptavidin-HRP (Zymed) at a 1:2500 dilution in PBS-0.05% Tween, 0.1% BSA at room temp for lhr. The plates were washed one last time and developed with 100 µl/well TMB substrate (3,3',5,5' - tetramethylbenzidine, Kirkegaard and Perry, Gaithersburg, MD) and the reaction stopped after color developed, with H₂SO₄, 50 µl/well. Absorbance (OD) were determined at 450 nm using 570 nm as a reference wavelength and the cytokine concentration evaluated using the standard curve.

### 6.2. RESULTS

### 6.2.1. TRI-FUSION PROTEINS INDUCED IMMUNE RESPONSES

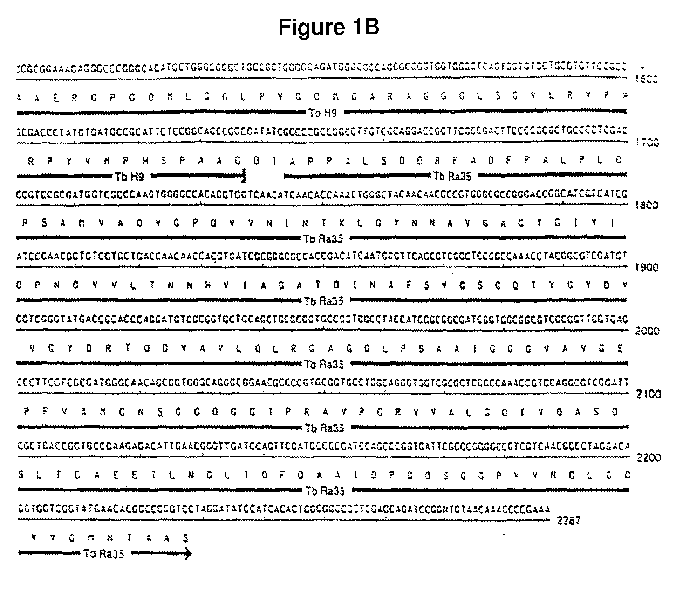

Three coding sequences for *M*. *tuberculosis* antigens were inserted into an expression vector for the production of a fusion protein. The antigens designated Ra12, TbH9 and Ra35 were produced as one recombinant fusion protein (Figure 1A and 1B). Antigens Erd14, DPV and MTI were produced as a second fusion protein (Figure 2). The two fusion proteins were affinity purified for use in *in vitro* and *in vivo* assays.

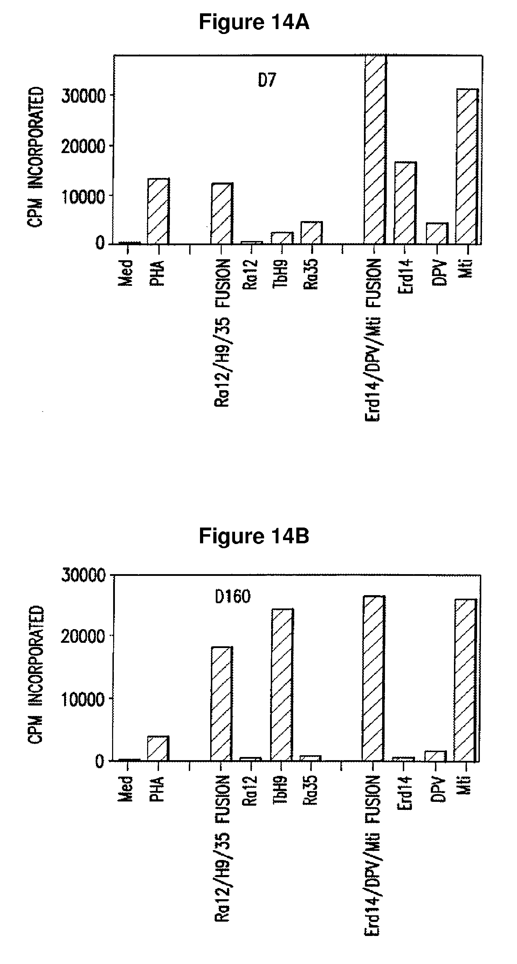

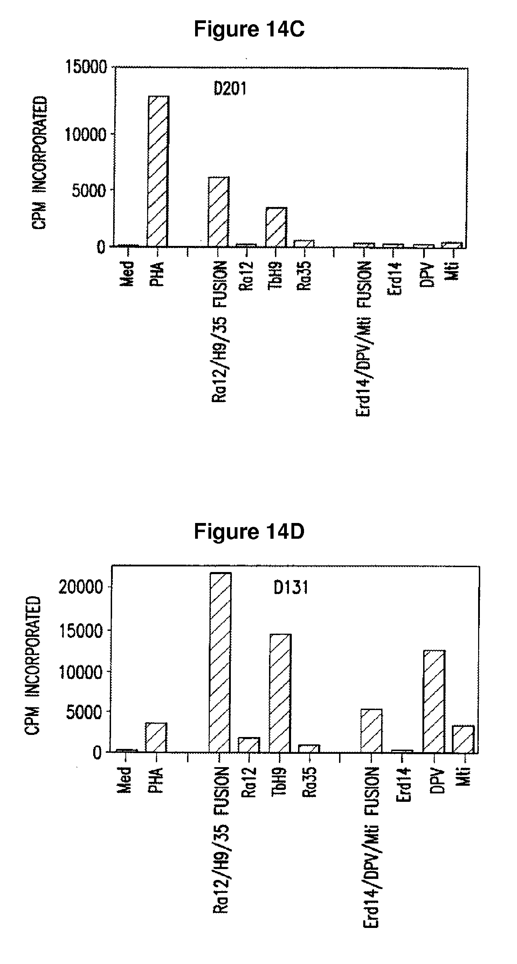

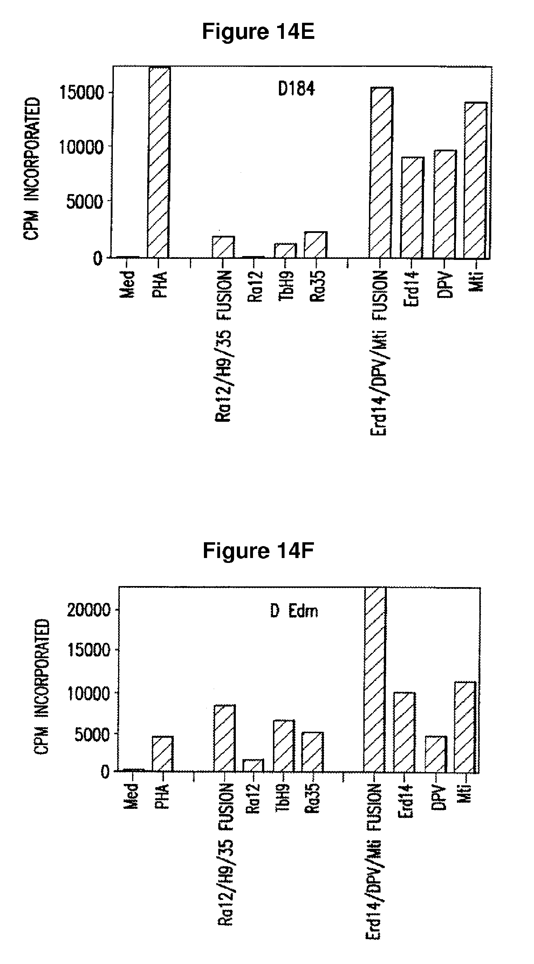

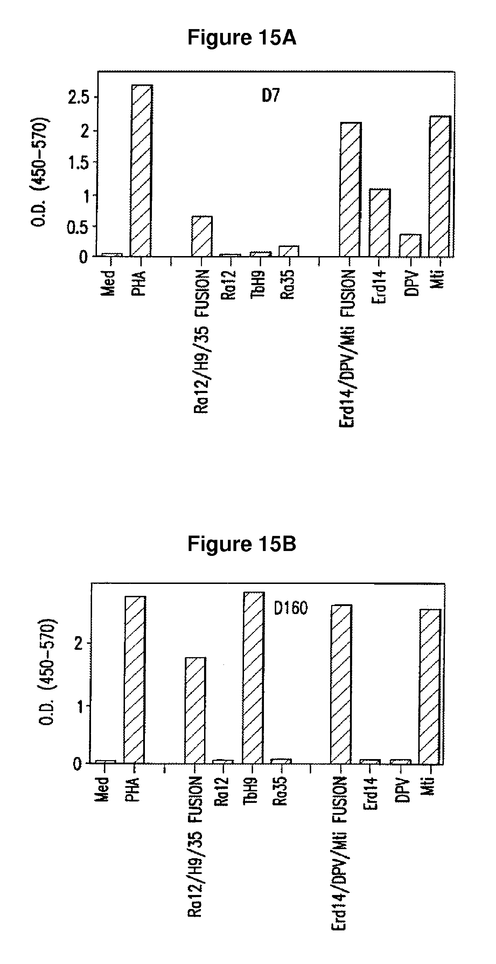

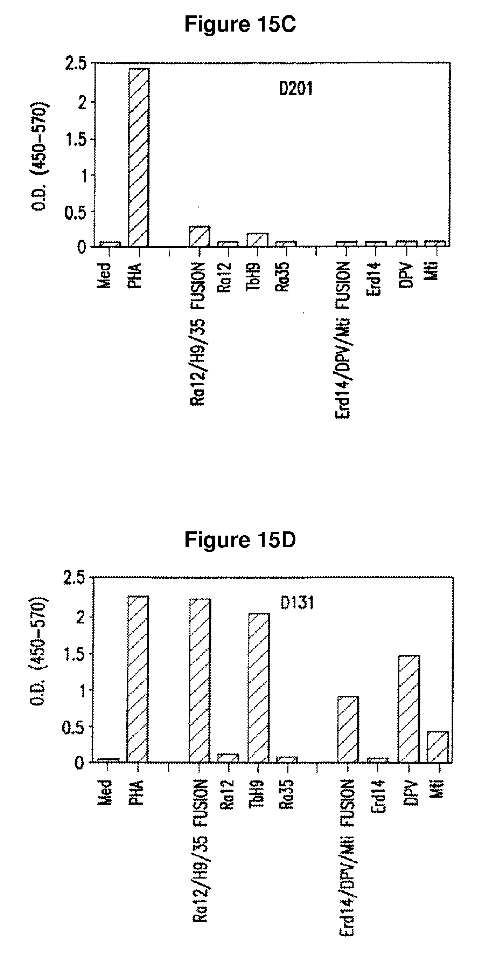

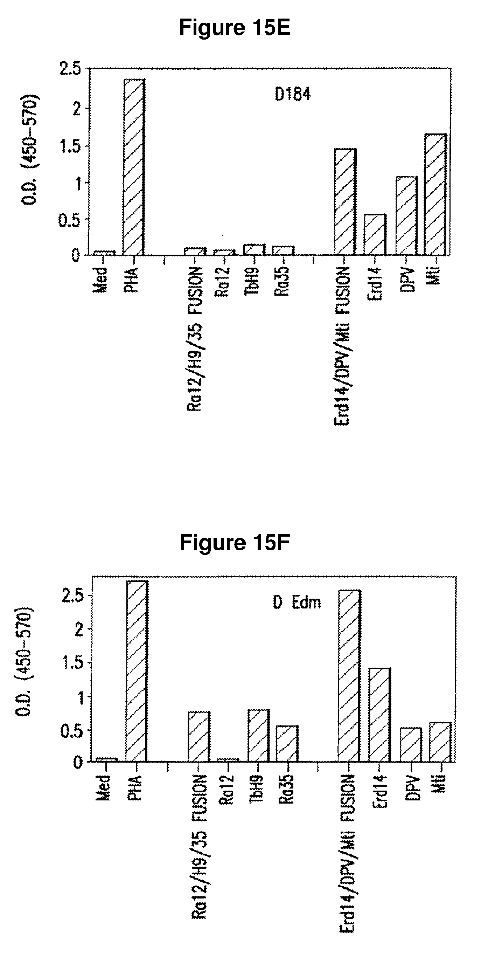

The two fusion proteins were tested for their ability to stimulate T cell responses from six PPD⁺ subjects. When T cell proliferation was measured, both fusion proteins exhibited a similar reactivity pattern as their individual components (Figure 14A-14F). A similar result was obtained when IFN-γ production was measured (Figure 15A-15F). For example, subject D160 responded to antigens TbH9 and MTI individually. Subject D160 also responded to the fusion proteins that contained these antigens (Figure 14B and 15B). In contrast, no T cell response from D160 was observed to other antigens individually. Another subject, D201, who did not react with antigens Erd14, DPV or MTI individually, was also unresponsive to the fusion protein containing these antigens. It should be noted that when the T cell responses to the individual components of the two fusion proteins were not particularly strong, the fusion proteins stimulated responses that were equal to or higher than that induced by the individual antigens in most cases.

The Ra12-TbH9-Ra35 tri-fusion protein was also tested as an immunogen *in vivo.* In these experiments, the fusion protein was injected into the footpads of mice for immunization. Each group of three mice received the protein in a different adjuvant formulation: SBAS1c, SBAS2 (Ling et al., 1997, Vaccine 15:1562-1567), SBAS7 and AL(OH)₃. After two subcutaneous immunizations at three week intervals, the animals were sacrificed one week later, and their draining lymph nodes were harvested for use as responder cells in T cell proliferation and cytokine production assays.

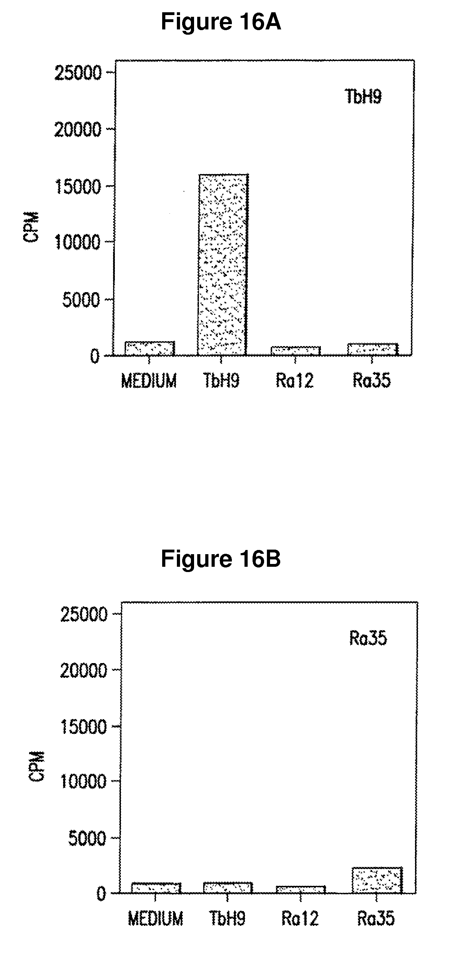

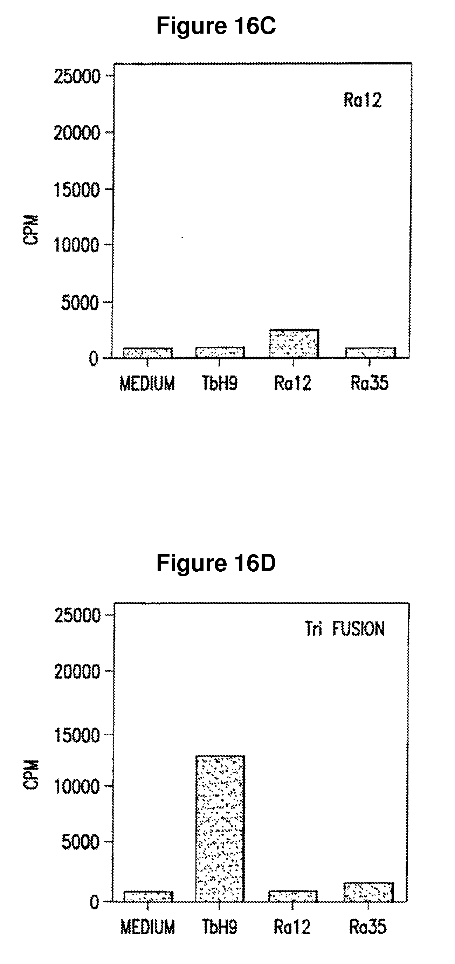

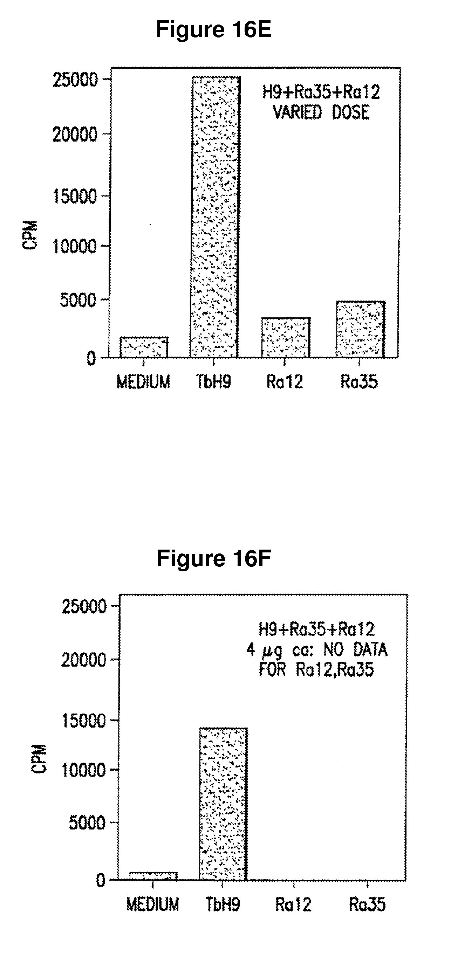

Regardless which adjuvant was used in the immunization, strong T cell proliferation responses were induced against TbH9 when it was used as an individual antigen (Figure 16A). Weaker responses were induced against Ra35 and Ra12 (Figure 16B and 16C). When the Ra12-TbH9-Ra35 fusion protein was used as immunogen, a response similar to that against the individual components was observed.

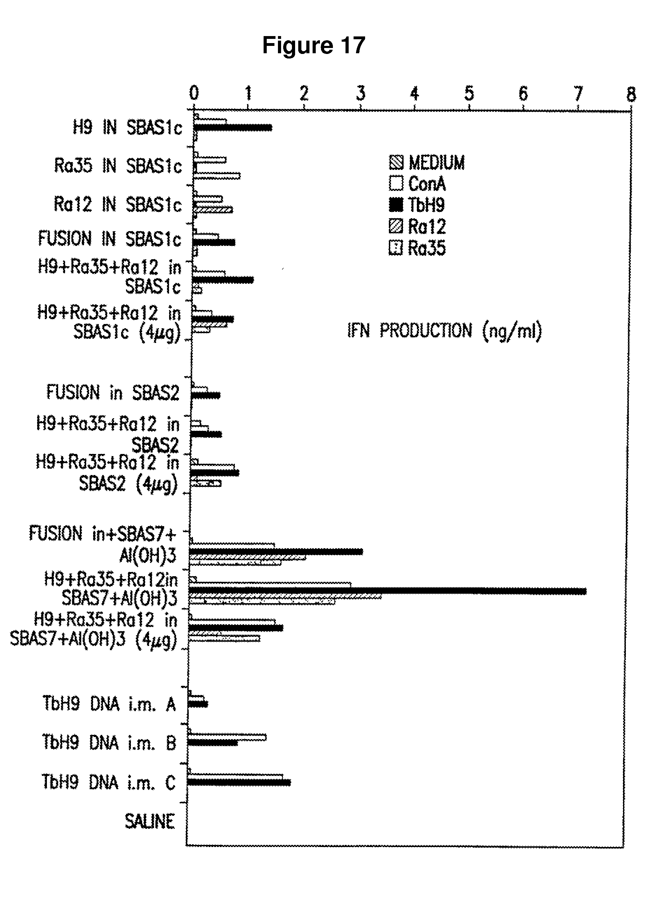

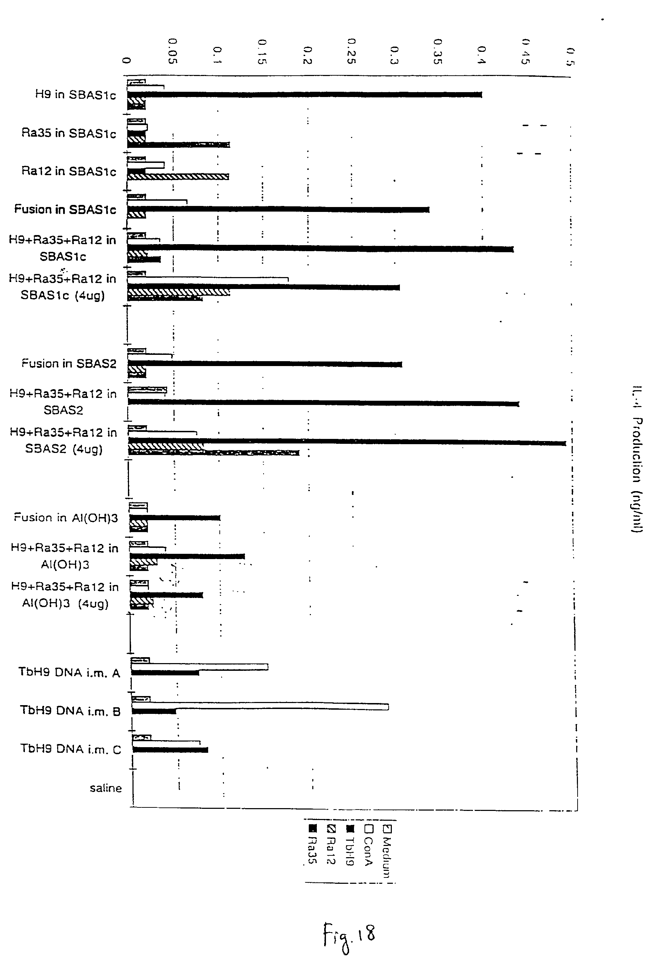

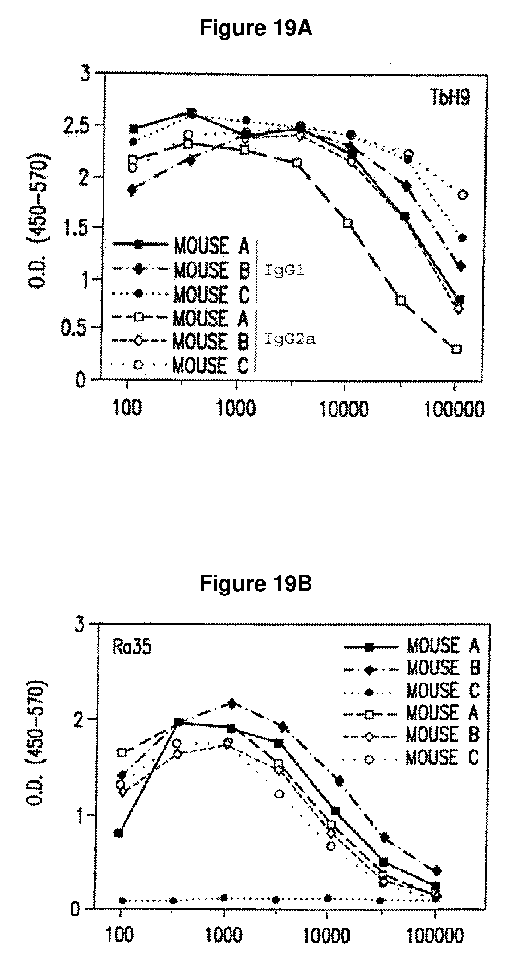

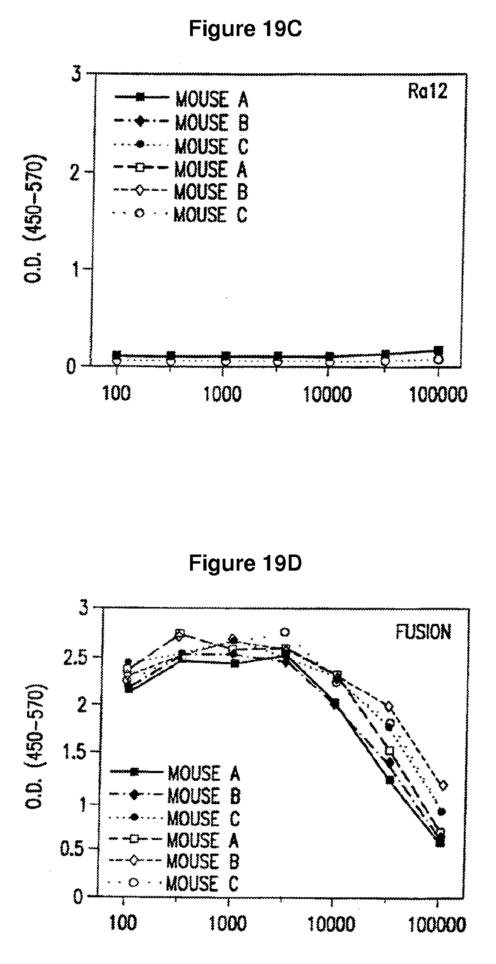

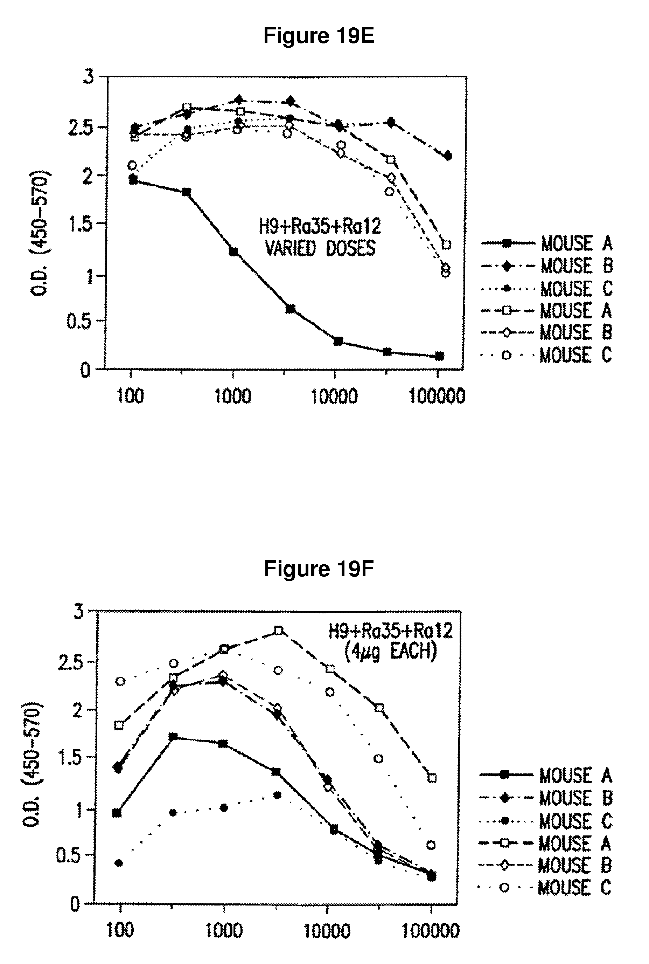

When cytokine production was measured, adjuvants SBAS1c and SBAS2 produced similar IFN-γ (Figure 17) and IL-4 responses (Figure 18). However, the combination of SBAS7 and aluminum hydroxide produced the strongest IFN-γ responses and the lowest level of IL-4 production for all three antigens. With respect to the humoral antibody response *in vivo*, Figure 19A-19F shows that the fusion protein elicited both IgG₁ and IgG₂ₐ antigen-specific responses when it was used with any of the three adjuvants.

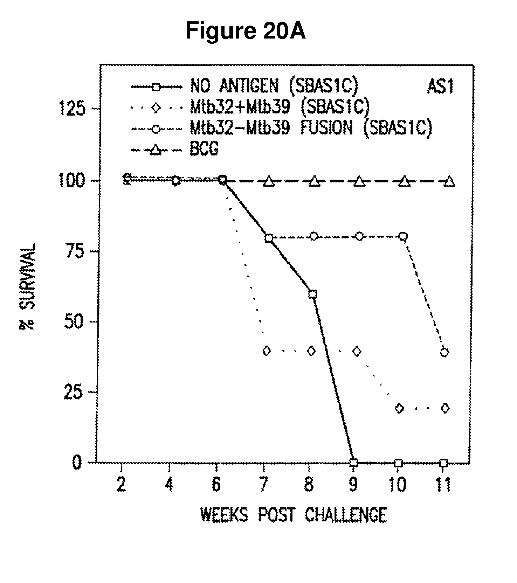

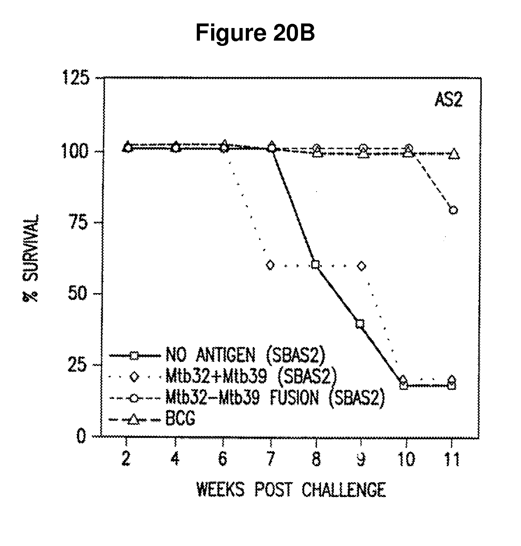

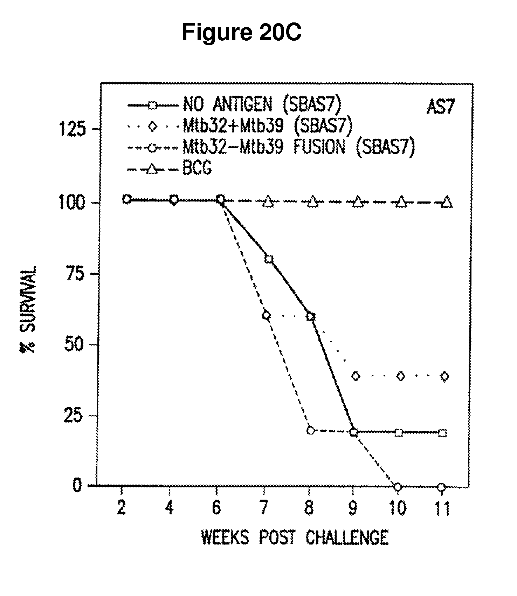

Additionally, C57BL/6 mice were immunized with a combination of two expression constructs each containing Ra12-TbH9-Ra35 (Mtb32A) or Erd14-DPV-MTI (Mtb39A) coding sequence as DNA vaccines. The immunized animals exhibited significant protection against tuberculosis upon a subsequent aerosol challenge of live bacteria. Based on these results, a fusion construct of Mtb32A and Mtb39A coding sequences was made, and its encoded product tested in a guinea pig long term protection model. In these studies, guinea pigs were immunized with a single recombinant fusion protein or a mixture of Mtb32A and Mtb39A proteins in formulations containing an adjuvant. Figure 20A-20C shows that guinea pigs immunized with the fusion protein in SBAS1c or SBAS2 were better protected against the development of tuberculosis upon subsequent challenge, as compared to animals immunized with the two antigens in a mixture in the same adjuvant formulation. The fusion proteins in SBAS2 formulation afforded the greatest protection in the animals. Thus, fusion proteins of various *M. tuberculosis* antigens may be used as more effective immunogens in vaccine formulations than a mixture of the individual components.

### 6.2.2. BI-FUSION PROTEIN INDUCED IMMUNE RESPONSES

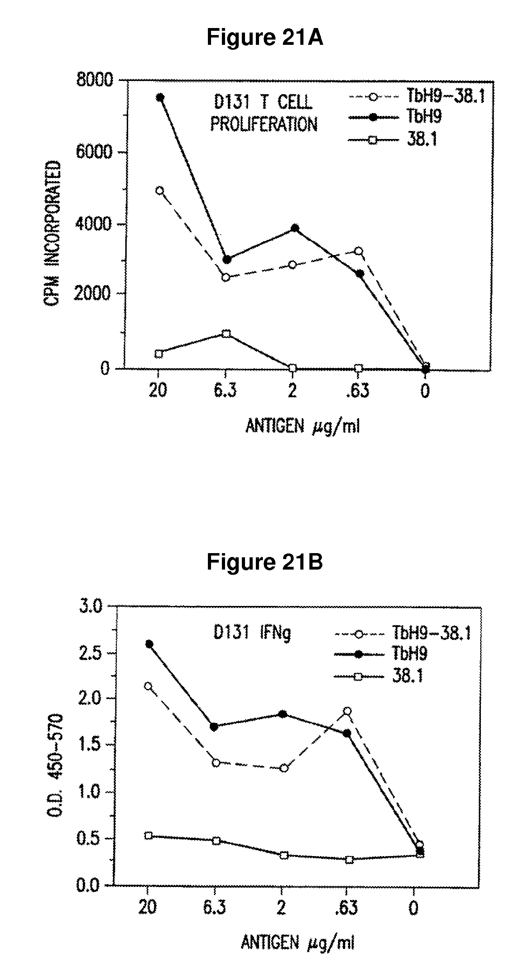

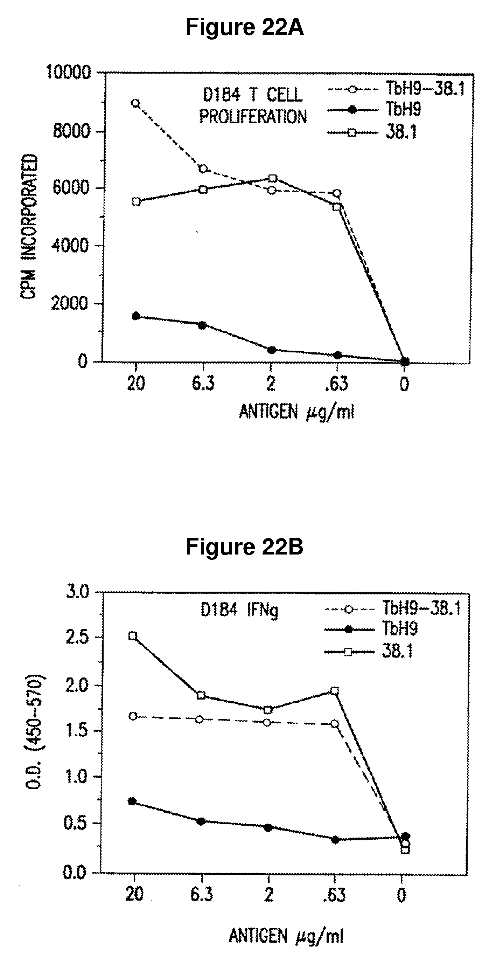

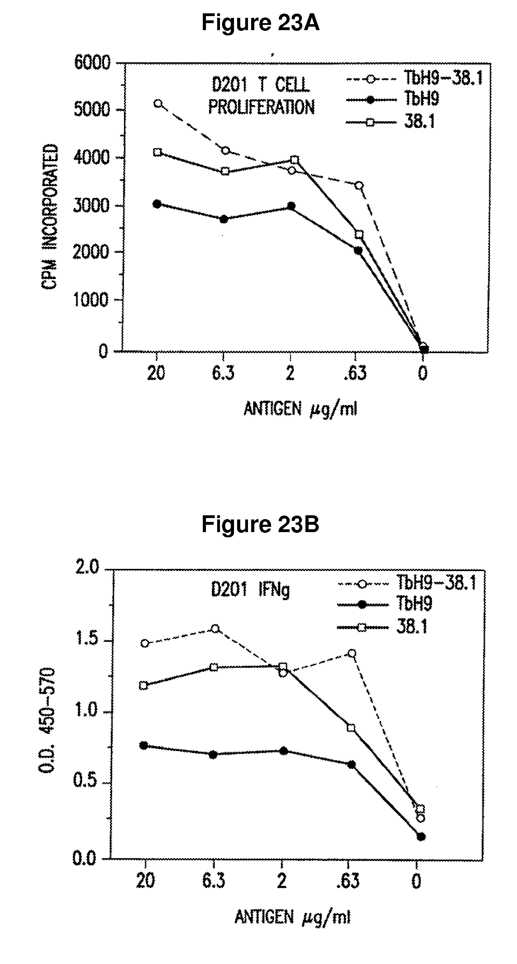

A bi-fusion fusion protein containing the TbH-9 and Tb38-1 antigens without a hinge sequence was produced by recombinant methods. The ability of the TbH9-Tb38-1 fusion protein to induce T cell proliferation and IFN-γ production was examined. PBMC from three donors were employed: one donor had been previously shown to respond to TbH9 but not to Tb38-1 (donor 131); one had been shown to respond to Tb38-1 but not to TbH9 (donor 184); and one had been shown to respond to both antigens (donor 201). The results of these studies demonstrate the functional activity of both the antigens in the fusion protein (Figures 21A and 21B, 22A and 22B, and 23A and 23B).

### 6.2.3. A TETRA-FUSION PROTEIN REACTED WITH TUBERCULOSIS PATIENT SERA

A fusion protein containing TbRa3, 38KD antigen, Tb38-1 and DPEP was produced by recombinant methods. The reactivity of this tetra-fusion protein referred to as TbF-2 with sera from *M. tuberculosis*-infected patients was examined by ELISA. The results of these studies (Table 1) demonstrate that all four antigens function independently in the fusion protein.

One of skill in the art will appreciate that the order of the individual antigens within each fusion protein may be changed and that comparable activity would be expected provided that each of the epitopes is still functionally available. In addition, truncated forms of the proteins containing active epitopes may be used in the construction of fusion proteins.

The present invention is not to be limited in scope by the exemplified embodiments which are intended as illustrations of single aspects of the invention, and any clones, nucleotide or amino acid sequences which are functionally equivalent are within the scope of the invention. Indeed, various modifications of the invention in addition to those described herein will become apparent to those skilled in the art from the foregoing description and accompanying drawings. Such modifications are intended to fall within the scope of the appended claims. It is also to be understood that all base pair sizes given for nucleotides are approximate and are used for purposes of description.

All publications cited herein are incorporated by reference in their entirety.

**TABLE 1**

| REACTIVITY OF TBF-2 FUSION PROTEIN WITH TB AND NORMAL SERA | | | | | | | | | |
|---|---|---|---|---|---|---|---|---|---|
| Serum ID | Status | TbF OD450 | Status | TbF-2 OD450 | Status | ELISA Reactivity | | | |
| | | | | | | 38 kD | TbRa3 | Tb38-1 | DPEP |
| B931-40 | TB | 0.57 | + | 0.321 | + | - | + | - | + |
| B931-41 | TB | 0.601 | + | 0.396 | + | + | + | + | - |
| B931-109 | TB | 0.494 | + | 0.404 | + | + | + | ±± | - |
| B931-132 | TB | 1.502 | + | 1.292 | + | + | + | + | ±± |
| 5004 | TB | 1.806 | + | 1.666 | + | ±± | ±± | + | - |
| 15004 | TB | 2.862 | + | 2.468 | + | + | + | + | - |
| 39004 | TB | 2.443 | + | 1.722 | + | + | + | + | - |
| 68004 | TB | 2.871 | + | 2.575 | + | + | + | + | - |
| 99004 | TB | 0.691 | + | 0.971 | + | - | ±± | + | - |
| 107004 | TB | 0.875 | + | 0.732 | + | - | ±± | + | - |
| 92004 | TB | 1.632 | + | 1.394 | + | + | ±± | ±± | - |
| 97004 | TB | 1.491 | + | 1.979 | + | + | ±± | - | + |
| 118004 | TB | 3.182 | + | 3.045 | + | + | ±± | - | - |
| 173004 | TB | 3.644 | + | 3.578 | + | + | + | + | - |
| 175004 | TB | 3.332 | + | 2.916 | + | + | + | - | - |
| 274004 | TB | 3.696 | + | 3.716 | + | - | + | - | + |
| 276004 | TB | 3.243 | + | 2.56 | + | - | - | + | - |
| 282004 | TB | 1.249 | + | 1.234 | + | + | - | - | - |
| 289004 | TB | 1.373 | + | 1.17 | + | - | + | - | - |
| 308004 | TB | 3.708 | + | 3.355 | + | - | - | + | - |
| 314004 | TB | 1.663 | + | 1.399 | + | - | - | + | - |
| 317004 | TB | 1.163 | + | 0.92 | + | + | - | - | - |
| 312004 | TB | 1.709 | + | 1.453 | + | - | + | - | - |
| 380004 | TB | 0.238 | - | 0.461 | + | - | ±± | - | + |
| 451004 | TB | 0.18 | - | 0.2 | - | - | - | - | ±± |
| 478004 | TB | 0.188 | - | 0.469 | + | - | - | - | ±± |
| 410004 | TB | 0.384 | + | 2.392 | + | ±± | - | - | + |
| 411004 | TB | 0.306 | + | 0.874 | + | - | + | - | + |
| 421004 | TB | 0.357 | + | 1.456 | + | - | + | - | + |
| 528004 | TB | 0.047 | - | 0.196 | - | - | - | - | + |
| A6-87 | Normal | 0.094 | - | 0.063 | - | - | - | - | - |
| A6-88 | Normal | 0.214 | - | 0.19 | - | - | - | - | - |
| A6-89 | Normal | 0.248 | - | 0.125 | - | - | - | - | - |
| A6-90 | Normal | 0.179 | - | 0.206 | - | - | - | - | - |
| A6-91 | Normal | 0.135 | - | 0.151 | - | - | - | - | - |
| A6-92 | Normal | 0.064 | - | 0.097 | - | - | - | - | - |
| A6-93 | Normal | 0.072 | - | 0.098 | - | - | - | - | - |
| A6-94 | Normal | 0.072 | - | 0.064 | - | - | - | - | - |
| A6-95 | Normal | 0.125 | - | 0.159 | - | - | - | - | - |
| A6-96 | Normal | 0.121 | - | 0.12 | - | - | - | - | - |
| | | | | | | | | | |
| Cut-off | | 0.284 | | 0.266 | | | | | |

## Claims

1. A polypeptide comprising an amino acid sequence encoded by a polynucleotide that hybridizes under moderately stringent conditions to a second polynucleotide which is complementary to a nucleotide sequence that encodes the amino acid of SEQ ID NO: 24 or 2, said amino acid sequence induces an immune response to *M. tuberculosis.*

2. A polypeptide according to claim 1, encoded by a polynucleotide that hybridizes under moderately stringent conditions to a second polynucleotide which is complementary to a nucleotide sequence that encodes the amino acid of SEQ ID NO: 24 or 2, said amino acid sequence induces an immune response to *M. tuberculosis.*

3. A polypeptide comprising the amino acid sequence of SEQ ID NO: 24 or 2, said amino acid sequence may optionally contain one or more conservative amino acid substitutions.

4. The polypeptide of Claim 1 or 2 which is a soluble polypeptide.

5. The polypeptide of Claim 1 or 2 which induces a T cell response.

6. The polypeptide of Claim 1 or 2 which is fused with a second heterologous polypeptide.

7. A pharmaceutical composition comprising the polypeptide of Claim 1 or 2.

8. A pharmaceutical composition comprising a polynucleotide that encodes the polypeptide of Claim 1 or 2.

9. A polypeptide according to claim 1 for use as a medicament.

10. A polypeptide according to claim 1 or 2 for use in the prevention of tuberculosis.

11. A polynucleotide which comprises a nucleic acid sequence which hybridizes under moderately stringent conditions to a second polynucleotide which is complementary to a nucleotide sequence that encodes the amino acid of SEQ ID NO: 24 or 2, said polynucleotide encoding an amino acid sequence which induces an immune response to *M. tuberculosis.*

12. An expression vector comprising a polynucleotide according to claim 11.

13. An expression vector according to claim 12 which is a viral vector.

14. A vaccine composition comprising a polypeptide according to claim 1 or 2 and an adjuvant.

15. A vaccine composition comprising a polynucleotide according to claim 11.

16. A polynucleotide according to claim 11 for use as a medicament.

17. A polynucleotide according to claim 11 for use in the prevention of tuberculosis.

18. A method for the production of a polypeptide according to claim 1 or 2 comprising recombinant expression of a polynucleotide according to claim 11.
